# EUROPÄISCHE PATENTSCHRIFT

(11) **EP 1 483 285 B1**
(45) Veröffentlichungstag und Bekanntmachung des Hinweises auf die Patenterteilung: **07.05.2008**
(21) Anmeldenummer: 03743869.4
(22) Anmeldetag: 11.03.2003
(51) Int. Cl.: C07K 5/06, A61K 31/435, A61K 38/04, A61P 7/02, C07C 257/00, C07D 213/34

(54) **HEMMSTOFFE DES GERINNUNGSFAKTORS Xa, IHRE HERSTELLUNG UND VERWENDUNG**
INHIBITORS OF THE BLOOD-CLOTTING FACTOR Xa, PRODUCTION THEREOF AND USE OF THE SAME
INHIBITEURS DU FACTEUR DE COAGULATION Xa, LEUR PRODUCTION ET LEUR UTILISATION

(30) Priorität: 11.03.2002 DE 10210590
(43) Veröffentlichungstag der Anmeldung: 08.12.2004
(73) Patentinhaber: Curacyte AG, 81675 München (DE)
(72) Erfinder: STÜRZEBECHER, Jörg, 99094 Erfurt (DE); STEINMETZER, Torsten, 07743 Jena (DE); SCHWEINITZ, Andrea, 07745 Jena (DE)
(74) Vertreter: Bösl, Raphael Konrad
(86) Internationale Anmeldenummer: PCT/EP2003/002487
(87) Internationale Veröffentlichungsnummer: WO 2003/076457

(56) Entgegenhaltungen:
- WO-A-00/58346
- WO-A-01/96366
- WO-A-94/29336
- US-A- 5 726 159
- US-A- 6 030 972
- HO J Z ET AL: "Exploratory Solid-Phase Synthesis Of factor Xa Inhibitors: DIscovery And Application of P3-Heterocyclic Amides As Novel Types Of Non-Basis Arginine Surrogates" BIOORGANIC & MEDICINAL CHEMISTRY LETTERS, OXFORD, GB, Bd. 9, 1999, Seiten 3459-3464, XP002245018 ISSN: 0960-894X in der Anmeldung erwähnt
- KUENZEL S ET AL: "4-Amidinobenzylamine-Based Inhibitors of Urokinase" BIOORGANIC & MEDICINAL CHEMISTRY LETTERS, OXFORD, GB, Bd. 12, 25. Februar 2002 (2002-02-25), Seiten 644-648, XP002245019 ISSN: 0960-894X

## Beschreibung

Die Erfindung betrifft neue Hemmstoffe des Gerinnungsfaktors Xa, ihre Herstellung und Verwendung zur Therapie, Prophylaxe und Diagnose von kardiovaskulären Erkrankungen und thromboembolischen Ereignissen.

Die gegenwärtig klinisch eingesetzten Antikoagulantien vom Heparin-Typ bzw. die Vitamin-K-Antagonisten werden nicht allen Anforderungen an ein "ideales" Antithrombotikum gerecht. Deshalb wird mit kleinmolekularen Hemmstoffen der Gerinnungsenzyme, speziell von Thrombin und Faktor Xa (F Xa), nach Alternativen gesucht. Ein besonderer Vorteil von F Xa-Hemmstoffen im Vergleich zu Thrombin-Hemmstoffen könnte die geringere Blutungsneigung sein, die sich bei verschiedenen Tierversuchen gezeigt hat. So wurde bei antithrombotisch effektiven Dosen die Blutungszeit nur minimal beeinflußt (J.M. Herbert et al., J. Pharmacol. Exp. Ther. 276, 1030-1038, 1996; K. Sato et al., Br. J. Pharmacol. 123, 92-96, 1998).

Die ersten nichtpeptidischen Verbindungen mit hoher Affinität für F Xa waren symmetrische Bis-benzamidine (Kᵢ = 13 nM für die wirksamste Verbindung BABCH) (J. Stürzebecher et al., Thromb. Res. 54, 245-252, 1998). Auch das Naphthamidin-Derivat DX-9065a besitzt zwei basische Gruppen und hemmt F Xa selektiv mit einem Kᵢ = 24 nM (T. Hara et al., Thromb. Haemost. 71, 314-319, 1994). Der mit DX-9065a strukturell verwandte Inhibitor YM-60828 (K. Sato et al. Eur. J. Pharmacol. 339, 141-146, 1997) ist noch wirksamer (Kᵢ = 1.3 nM). Inzwischen wurde eine ganze Reihe weiterer bis-basischer Verbindungen beschrieben, bei denen z. B. zwei Benzamidin-Reste über einen Oxazolin-Ring (Kᵢ = 18 nM) (M.L. Quan et al., Bioorg. Med. Chem. Lett. 7, 2813-2818, 1997) bzw. eine Carboxymethylalkyl-Kette (Kᵢ = 34 nM) verknüpft sind (T.P. Maduskuie et al., J. Med. Chem. 41, 53-62, 1998). Nachteil der bis-basischen Verbindungen ist insbesondere die geringe Bioverfügbarkeit nach oraler Gabe.

Auch Hemmstoffe für F Xa, die nur eine basische Gruppe enthalten, wurden beschrieben. N-substituierte Amidino-phenoxypyridine (Kᵢ = 0,11 nM für BX-807834) wurden auf der Basis von BABCH entwickelt (R. Mohan et al., Bioorg. Med. Chem. Lett. 8, 1877-1882, 1998; G.B. Phillips et al. J. Med. Chem. 41, 3557-3562, 1998). Amide des Nα-Adamantyloxycarbonyl-3-amidinophenylalanins (Kᵢ = 74 nM für die wirksamste Verbindung) sind selektive Hemmstoffe des F Xa (S. Sperl et al., Biol. Chem. 381, 321-329, 2000), während Nα-arylsulfonyl-aminoacylierte Ester des 3-Amidinophenylalanins eine geringe Hemmwirkung (Kᵢ = 840 nM für TAPAM) besitzen (J. Stürzebecher et al., Thromb. Res. 54, 245-252, 1998). Die WO 96/10022 offenbart Hemmstoffe, die überhaupt keine starke Ladung mehr besitzen (Kᵢ = 3,0 nM für die wirksamste Verbindung).

Bisher wurden nur wenige Peptide als Hemmstoffe für F Xa beschrieben, die sich von der Substrat-Sequenz Ile-Glu-Gly-Arg ableiten. Die von Kettner und Shaw (Thromb. Res. 22, 645-652, 1981) beschriebenen Chlormethylketone hemmen F Xa irreversibel und sind nicht für in vivo-Anwendungen geeignet. Dagegen sind die Peptide SEL 2489 (Kᵢ = 25 nM) und SEL 2711 (Kᵢ = 3 nM) außerordentlich wirksam (J. A. Ostrem et al., Biochemistry 37, 1053-1059, 1998). Auch einige Peptidyl-Arginin-Aldehyde und Peptidyl-Arginyl-Ketone wurden beschrieben, die neben Argininal oder einem Arginyl-Ketonderivat, wie z.B. Arginyl-Ketothiazol in P3-Position ein D-Arginin bzw. eine unnatürliche basische Aminosäure, wie z.B. 4-Amidinophenylalanin, 3- oder 4- Amidinopiperidinylalanin und 4-Guanidinophenylalanin in P3 besitzen (Z. H. Jonathan, Bioorg. Med. Lett. 9, 3459-3464, 1999 und Übersichtsarbeit: Zhu und Scarborough Current Opinion in Cardiovascular, Pulmonary & Renal Investigational Drugs 1999, 1, 63-88).) In der Anmeldung WO 01/96366 sind Hemmstoffe offenbart, die sich von acyliertem Amidinobenzylamin ableiten und neben einer natürlichen Aminosäure in P2 einen D-Ser-Ether oder ein vergleichbares Derivat einer unnatürlichen Aminosäure enthalten. Verbindungen diese Typs hemmen sowohl F Xa (Kᵢ = 30 nM für die wirksamste Verbindung) als auch die Gerinnung von menschlichem Blutplasma sehr wirksam. Allerdings haben Verbindungen diese Typs nur unzureichende pharmakokinetische Eigenschaften für eine Anwendung in vivo; sie werden nach oraler Gabe kaum resorbiert und im Versuchstier nach i.v.-Gabe sehr schnell aus der Zirkulation eliminiert.

Der Erfindung liegt daher die Aufgabe zu Grunde, einen auch für therapeutische Anwendungen geeigneten Wirkstoff anzugeben, der den Gerinnungsfaktor Xa mit hoher Aktivität und Spezifität hemmt und der nach i.v.-, s.c.- oder oraler Gabe möglichst lange im Körper zirkuliert.

Überraschend wurde gefunden, dass acyliertes Amidinobenzylamin gemäß der im Patentanspruch 1 angeführten allgemeinen Formel I, 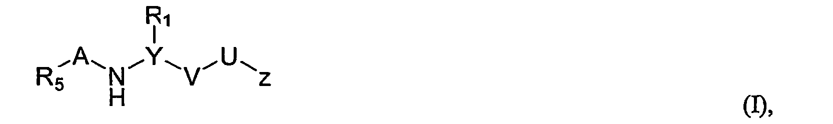 wobei
A P₂-P₁ mit 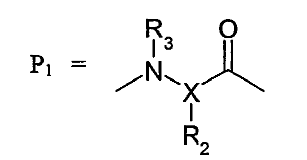 und
P₂ von einer der folgenden Aminosäuren in der D-Konfiguration abstammt: D-2,3-Diaminopropionsäure, D-2,4-Diaminobuttersäure, D-Citrullin, D-Homocitrullin, D-Norcitrullin, D-Arginin, D-Homoarginin, D-Norarginin, D-3-Guanidinophenylalanin, D-3-Guanidinophenylhomoalanin, D-3-Guanidinophenylglycin, D-3-Amidinophenylalanin, D-3-Amidinophenylhomoalanin, D-3-Amidinophenylglycin, D-4-Guanidinomethylphenylalanin, D-4-Guanidinomethylphenylhomoalanin, D-4-Guanidinomethylphenylglycin, D-3-Guanidinomethylphenylalanin, D-3-Guanidinomethylphenylhomoalanin, D-3-Guanidinomethylphenylglycin, D-3-Amidinopiperidinylalanin, D-3-Amidinopiperidinylhomoalanin, D-3-Amidinopiperidinylglycin, n-Butylamidinoglycin, n-Pentylamidinoglycin, n-Propylamidinoglycin, D-Canavanin, D-Homocanavanin, D-Norcanavanin, 2-Amino-4-Amidinohydrazono-Buttersäure, 2-Amino-3-Amidinohydrazono-Propionsäure, 2-Amino-5-Amidinohydrazono-Pentansäure, 2-Amino-4-(Pyridin-4-ylamino)-Buttersäure, 2-Amino-4-(Pyridin-4-ylatnino)-Propionsäure, 2-Amino-4-(Pyridin-4-ylamino)-Pentansäure, 4-Imidazoly-Propargylglycin, D-Histidin, D-Homohistidin, D-Histidin-(1-Methyl), D-Homohistidin-(1-Methyl), D-Histidin-(3-Methyl), D-Homohistidin-(3-Methyl), D-Alanin(4-[5-2(-amino)imidazoyl], D-Homoalanin(4-[5-2(-amino)imidazoyl], D-Glycin(4-[5-2(-amino)imidazoyl], D-Alanin(3-(2-Pyrimidinyl), D-Homoalanin(3-(2-Pyrimidinyl), D-Alanin(3-(5-Pyrimidinyl), D-Homoalanin(3-(5-Pyrimidinyl), D-2-Amino-3-(2-amino-pyrimidin-5-yl)-propionsäure, D-2-Amino-4-(2-amino-pyrimidin-5-yl)-buttersäure, D-Alanin(3-(2-benzimidazolyl)), D-Homoalanin(3-(2-benzimidazolyl)), D-Tryptophan das mit Aminoalkylgruppen am Indolring substituiert ist, D-Homotryptophan das mit Aminoalkylgruppen am Indolring substituiert ist, D-2-Amino-3-(6-amino-pyridin-3-yl)-propionsäure, D-2-Amino-4-(6-amino-pyridin-3-yl)-buttersäure, D-2-Amino-3-(6-amino-2-methyl-pyridin-3-yl)-propionsäure, D-2-Amino-4-(6-amino-2-methyl-pyridin-3-yl)-buttersäure, D-2-Amino-3-(6-amino-2,4-dimethyl-pyridin-3-yl)-propionsäure, D-2-Amino-4-(6-amino-2,4-dimethyl-pyridin-3-yl)-buttersäure, D-3-Hydroxyamidinophenylalanin, D-3-Hydroxy-amidinophenylhomoalanin, D-3-Hydroxyamidinophenylglycin
bei denen X, R₂ und R₃ natürliche und/oder unnatürliche Aminosäuren ergeben, dann sowohl Faktor Xa sehr wirksam inaktivieren als auch langsam aus der Zirkulation eliminiert
werden, wenn neben der Amidinofunktion weitere geladene Gruppen, vorzugsweise Carboxyl, Amino, Amidino, Hydroxyamidino, Amidrazono oder Guanidino eingeführt werden. Die Carboxylgruppen können auch geschützt in Form ihrer Ester vorliegen, wobei bevorzugt Ethylester Verwendung finden. Diese Ester werden in vivo teilweise in die freien Säuren umgewandelt.

Die Benennung der Reste P₂ und P₁ in dem Struktursegment A der allgemeinen Formel I bezieht sich nicht auf die sonst üblicherweise verwendete Nomenklatur der Aminosäurereste in Peptidsubstraten von Serinproteasen und davon abgeleiteten Inhibitoren, wie sie von Schechter und Berger eingeführt wurde (Schechter und Berger, Biochem. Biophys. Res. Comm. 27, 157-162 (1967)). In allen Teilen der Erfindung, d.h. sowohl in der Beschreibung als auch in den Ansprüchen gelten die folgenden Definitionen:

Der Buchstabe P in Zusammenhang mit einer Zahl von 1 bis 3 in normaler Schrift, d.h. P1, P2 oder P3, wird für Aminosäurereste und deren Derivate entsprechend der Nomenklatur von Schechter und Berger verwendet. Dagegen steht der Buchstabe P in Zusammenhang mit einer tiefgestellten 1 oder 2, d.h. P₁ oder P₂, für Aminosäurereste und deren Derivate als Bestandteile der Struktur A in Formel I der vorliegenden Erfindung. Dabei entspricht substituierte oder unsubstituierte natürliche oder unatürliche Aminosäure P₁ in der Struktur A P2 nach Schechter und Berger und die in der D-Konfiguration vorliegende substituierte oder unsubstituierte natürliche oder unatürliche Aminosäure P₂ in der Struktur A entspricht P3 nach Schechter und Berger.

In Formel I ist
R₁ ein H oder -(CH₂)ₐCOOR₆ mit a = 0, 1, 2, 3, 4 oder 5, vorzugsweise mit a= 0, 1 oder 2, wobei R₆ ein verzweigter oder unverzweigter Alkylrest mit vorzugsweise 1 bis 6 C-Atomen, insbesondere 1 bis 3 C-Atomen, vor allem Ethyl, ist;
R₂ ist ein H, ein verzweigter oder unverzweigter Alkylrest mit 1 bis 8 C-Atomen, vorzugsweise mit 1 bis 3 C-Atomen, oder
-(CH₂)_{c}COOR₈ mit c = 1, 2, 3 oder 4, wobei R₈ H oder ein verzweigter oder unverzweigter Alkylrest mit vorzugsweise 1 bis 6 C-Atomen, insbesondere 1 bis 3 C-Atomen, vor allem Ethyl, ist, oder
-(CH₂)_{d}-OR₉ mit d =1, 2, 3 oder 4, wobei R₉ H ist, oder
-(CH₂)ₑOR₁₀, -(CH₂)ₑSR₁₀, -(CH₂)ₑ-Guanidino, -(CH₂)ₑ-Imidazol oder -(CH₂)ₑNHR₁₀ mit e = 1, 2, 3, 4 oder 5 ist, wobei R₁₀ H, ein verzweigter oder unverzweigter Alkylrest mit 1-16, insbesondere 1-8, vor allem 1-3 C-Atomen oder ein substituierter oder unsubstituierter Aryl-, Heteroaryl-, Aralkyl- oder Heteroaralkylrest ist, wobei der Alkylrest vorzugsweise 1 bis 16, insbesondere 1 bis 8, vor allem 1 bis 3 C-Atome, und der Aryl- oder Heteroarylrest vorzugsweise 4 bis 14, insbesondere 6 bis 10, vor allem 6 C-Atome und vorzugsweise 1 bis 3 N als Heteroatom besitzt;
R₃ ist ein H oder -(CH₂)_{b}R₇ mit b = 1, 2, 3, 4, 5, 6, 7 oder 8, vorzugsweise mit b = 2 oder 3, wobei R₇ H, ein verzweigter oder unverzweigter Alkylrest mit 1 bis 10 C-Atomen, vorzugsweise mit 1 bis 3 C-Atomen, oder ein geladener Rest ist;
R₅ ist -(CH₂)_{g}(CH₃)ₕ, -(CH₂)ᵢ-Aryl mit g + h = i = 0, 1, 2 oder 3, -SO₂R₁₂, -COR₁₂, oder -COOR₁₂, wobei R₁₂ ein verzweigtes oder unverzweigtes Alkyl mit 1-16, vorzugsweise 1 bis 8, insbesondere 1-4, vor allem 1-2 C-Atomen, ein substituierter oder unsubstituierter Aryl-, Heteroaryl-, Aralkyl- oder Heteroaralkylrest, ein Adamantyl-, ein Campher-, ein Cyclohexylmethylrest, vorzugsweise Benzyl, ist, wobei R₅ mit einer geladenen oder ungeladenen Gruppe ist;
U ist ein Phenyl- oder Cyclohexylrest; ein Heterophenyl- oder Heterocyclohexylrest mit vorzugsweise mindestens einem N, S oder O als Heteroatom, insbesondere Pyridin, Piperidin oder Pyrimidin oder ein Thiophenrest;
V ist (CH₂)ₙ mit n = 0, 1, 2 oder 3, vorzugsweise 0;
X ist N oder CH, vorzugsweise CH;
Y ist N oder (CH)ₘ mit m = 0 oder 1, vorzugsweise CH;
Z kommt in 3- oder 4-Position vor und ist eine Aminomethyl-, eine Guanidinofunktion oder eine Amidinogruppe 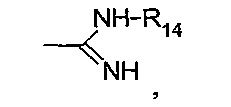 wobei R₁₄ H, OH, NH₂, -COR₁₅ oder -COOR₁₅ ist, wobei R₁₅ ein verzweigter oder unverzweigter Alkylrest mit 1 bis 16, vorzugsweise 1 bis 8, insbesondere 1 bis 4, vor allem 1 bis 2 C-Atomen oder ein substituierter oder unsubstituierter Aryl-oder Heteroaryl-, Aralkyl- oder Heteroaralkylrest ist, wobei der Alkylrest vorzugsweise 1 bis 16, insbesondere 1 bis 8, vor allem 1 bis 4 und besonders bevorzugt 1 bis 2 C-Atome und der Aryl- oder Heteroarylrest vorzugsweise 4 bis 14, insbesondere 6 bis 10, vor allem 6 C-Atome und vorzugsweise 1 bis 3 N als Heteroatom besitzt;
oder eine Verbindung mit
R₅ gleich 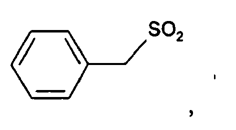 P₁ gleich 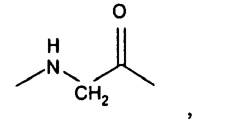 NH-YR₁-V-U-Z gleich 4-Amidinobenzylamid und P₂ ausgewählt aus D-Phe(3-Amidino) oder D-Arg;
dadurch gekennzeichnet, dass ein oder mehrere geladene Reste in den Resten R₁, R₂, R₃ oder R₅ vorhanden sind.
wobei ein oder mehrere geladene Reste vorzugsweise abgeleitet von -COOH, -CH(COOH)₂, -SO₂H, NH₂, einer Amidino-, Hydroxyamidino-, Amidrazono-oder Guanidinogruppe in den Resten R₁, R₂, R₃ oder R₅ vorhanden sind;
oder eine Verbindung der allgemeinen Formel I in Form eines Prodrugs oder in Form ihres Salzes.

Weitere besonders geeignete Verbindungen sind Verbindungen nach der allgemeinen Formel I, wobei U an 1, 2 oder 3 Positionen vorzugsweise mit einem Halogen, insbesondere Fluor oder Chlor, oder einem Methyl-, Ethyl-, Propyl-, Methoxy-, Ethoxy-, oder Propoxyrest substituiert ist.

Weitere besonders geeignete Verbindungen sind Verbindungen nach der allgemeinen Formel I, wobei eine Carboxylgruppe als Ester, bevorzugt als Ethylester, geschützt vorliegt.

Weitere besonders geeignete Verbindungen sind Verbindungen nach der allgemeinen Formel I, wobei R₉ in diesem Fall ein Alkylcarbonyl-, Aralkylcarbonyl-, Alkyloxycarbonyl- oder Aralkyloxycarbonyl-Rest ist, wobei der Alkylrest vorzugsweise 1 bis 6, insbesondere 1 bis 4 C-Atome und der Arylrest vorzugsweise 5 bis 8, insbesondere 6 C-Atome besitzt.

Weitere besonders geeignete Verbindungen sind Verbindungen nach der allgemeinen Formel I, wobei P₂ in der Struktur A der allgemeinen Formel I ein Arginin-Derivat ist, die eine geringe Basizität aufweist. Besonders geeignete Beispiele hierfür sind: D-Canavanin, D-Homocanavanin, D-Norcanavanin; D-Canavanin wird analog der Vorschrift für L-Canavanin mit D-Homoserin als Ausgangsstoff synthetisiert (Kim et al., Med. Chem. Res. 377-383 (1996). Weitere Beispiele sind: 2-Amino-4-Amidinohydrazono-Buttersäure, 2-Amino-5-Amidinohydrazono-Piopionsäure, 2-Amino-5-Amidinohydrazono-Pentansäure. Die Synthese erfolgt nach der Strategie, die für die Einführung der Amidrazonogruppe in eine Serie von Thrombin-hemmstoffen beschrieben wurde (Soll et al., Bioorg. Med. Chem. Lett. 10 (2000) 1-4). Des Weiteren 2-Amino-4-(Pyridin-4-ylamino)-Buttersäure, 2-Amino-4-(Pyridin-4-ylamino)-Propionsäure, 2-Amino-4-(Pyridin-4-ylamino)-Pentansäure, wobei die Synthese der Aminopyridinderivate wie in: von der Saal, Bioorg. Med. Chem. Lett. 7,1283-1288 (1997) beschrieben erfolgt. Ein weiteres Beispiel ist das 4-Imidazolyl-Propargylglycin, das aus Propargylglycin (Advanced Chemtech) und Pd-Katalysierter Kopplung mit N-Trityl-4-lodimidazol analog folgender Referenzen: Lee et al., Bioorg. Med. Chem. Lett. 10, 2775-2778 (2000); Kirk, K.I. J. Heterocycl. Chem. 22, 57 ff. (1985) hergestellt wird. Weitere Beispiele sind: D-Histidin, D-Homohistidin, D-Histidin-(1-Methyl), D-Homohistidin-(1-Methyl), D-Histidin-(3-Methyl), D-Homohistidin-(3-Methyl), D-Alanin(4-[5-2(-amino)imidazoyl], D-Homoalanin(4-[5-2(-amino)imidazoyl], D-Glycin(4-[5-2(-amino)imidazoyl], D-Alanin(3-(2-Pyrimidinyl), D-Homoalanin(3-(2-Pyrimidinyl), D-Alanin(3-(5-Pyrimidinyl), D-Homoalanin(3-(5-Pyrimidinyl), D-2-Amino-3-(2-aminopyrimidin-5-yl)-propionsäure, D-2-Amino-4-(2-amino-pyrimidin-5-yl)-buttersäure, D-Alanin(3-(2-benzimidazolyl)), D-Homoalanin(3-(2-benzimidazolyl)), D-Tryptophan das mit Aminoalkylgruppen am Indolring substituiert ist, D-Homotryptophan das mit Aminoalkylgruppen am Indolring substituiert ist, D-2-Amino-3-(6-amino-pyridin-3-yl)-propionsäure, D-2-Amino-4-(6-amino-pyridin-3-yl)-buttersäure, D-2-Amino-3-(6-amino-2-methyl-pyridin-3-yl)-propionsäure, D-2-Amino-4-(6-amino-2-methyl-pyridin-3-yl)-buttersäure, D-2-Amino-3-(6-amino-2,4-dimethyl-pyridin-3-yl)-propionsäure, D-2-Amino-4-(6-amino-2,4-dimethyl-pyridin-3-yl)-buttersäure, D-Citrullin, D-Homocitrullin, D-Norcitrullin, D-3-Hydroxyamidinophenylalanin, D-3-Hydroxyamidinophenylhomoalanin oder D-3-Hydroxyamidinophenylglycin. Ein Vorteil von Faktor Xa-Inhibitoren mit diesen weniger basischen D-Arginin-Mimetika besteht darin, dass sie bei physiologischem pH Wert nur teilweise geladen sind und daher oral besser aufgenommen werden.

Weitere besonders geeignete Verbindungen sind Verbindungen nach der allgemeinen Formel I, wobei die Verbindung die folgende Struktur aufweist 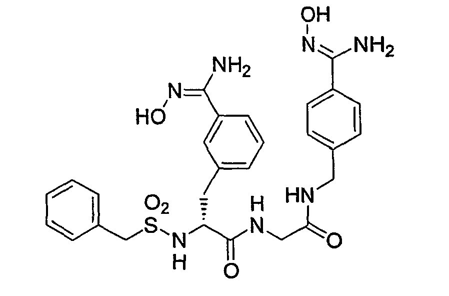 wobei die in der Struktur enthaltenen Hydroxamidinogruppen im Sinne eines Prodrugs erst nach Aufnahme im Körper in die analogen Amidinogruppen umgewandelt werden, wodurch die inhibitorisch wirksame Inhibitorstruktur entsteht.

Weitere besonders geeignete Verbindungen sind Verbindungen nach der allgemeinen Formel I, wobei der Substituent am substituierten Aryl-, Heteroaryl-, Aralkyl- oder Heteroaralkylrest ein Halogen, vorzugsweise Fluor, Chlor oder Brom, insbesondere Fluor oder Chlor, ist.

Neben der Inaktivierung von Faktor Xa werden die zusätzlich geladenen 4-Amidinobenzylamin-Derivate auf vorteilhafte und überraschende Weise sehr langsam eliminiert, so dass die erfindungsgemäßen Verbindungen eine neue Gruppe von hochaktiven F Xa-Hemmstoffen darstellen.

Die Verbindungen liegen in der Regel als Salze, vorzugsweise mit Mineralsäuren, bevorzugt als Hydrochloride, vor oder vorzugsweise als Salze mit geeigneten organischen Säuren. Bevorzugte Salze von Mineralsäuren sind auch Sulfate. Geeignete organische Säuren sind beispielsweise Essigsäure, Ameisensäure, Methylsulfonsäure, Bernsteinsäure, Apfelsäure oder Trifluoressigsäure, wobei bevorzugte Salze von organischen Säuren Acetate sind.

Die Verbindungen der allgemeinen Formel I können in prinzipiell bekannter Weise, wie nachfolgend beschrieben, beispielsweise wie folgt hergestellt werden:

Aus dem kommerziell erhältlichen 4-Cyanobenzylamin (Showa Denko, Japan) wird das Boc-geschützte 4-Acetyloxamidinobenzylamin nach dem Fachmann bekannten Verfahren gewonnen. Nach Abspaltung der Boc-Schutzgruppe erfolgt die Ankopplung der weiteren Aminosäuren und der Schutzgruppe R₅ mittels Standardkopplungsmethoden mit Boc als N-terminaler Schutzgruppe. Die zweite Aminosäure kann auch direkt als N-aryl- bzw. N-aralkyl-sulfonyl-geschützte Aminosäure gekoppelt werden. Die Peptidanaloga werden sequentiell, beginnend vom Acetyloxamidinobenzylamin, aufgebaut. Die meisten Zwischenprodukte kristallisieren gut und lassen sich damit einfach reinigen. Die Endreinigung der Hemmstoffe erfolgt auf der letzten Stufe vorzugsweise über präparative, reversed-phase HPLC.

Ein weiterer Gegenstand der Erfindung ist ein Verfahren zur Herstellung einer Verbindung nach der allgemeinen Formel I, wobei sequentiell die entsprechenden Aminosäuren an ein 4-Acetyloxamidinobenzylamin angekoppelt werden, wobei die N-terminale Aminosäure entweder den R₅-Rest bereits trägt oder dieser anschließend daran gebunden wird.

Ein weiterer Gegenstand der Erfindung ist ein Arzneimittel enthaltend einen erfindungsgemäßen Hemmstoff sowie weitere pharmazeutisch geeignete Hilfs-und/oder Zusatzstoffe. Geeignete Hilfs- und/oder Zusatzstoffe, die z.B. der Stabilisierung und/oder Konservierung des Arzneimittels dienen, sind dem Fachmann allgemein geläufig (z.B. Sucker H. et al., (1991) Pharmazeutische Technologie, 2. Auflage, Georg Thieme Verlag, Stuttgart). Hierzu zählen beispielsweise physiologische Kochsalzlösungen, Ringer-Dextrose, Ringer-Laktat, entmineralisiertes Wasser, Stabilisatoren, Antioxidantien, Komplexbildner, antimikrobielle Verbindungen, Proteinaseinhibitoren und/oder inerte Gase.

Das Arzneimittel könnte beispielsweise in parenteraler Anwendungsform, insbesondere in intraartieller, intravenöser, intramuskulärer oder subcutaner Form, in enteraler Anwendungsform, insbesondere zur oralen oder rektalen Anwendung, oder in topischer Anwendungsform, insbesondere als Dermatikum, angewendet werden. Bevorzugt sind intravenöse oder subkutane Anwendungen.

In einer Ausführungsform der Erfindung wird das Arzneimittel beispielsweise in Form einer Tablette, eines Dragees, einer Kapsel, eines Pellets, Suppositoriums, einer Lösung, insbesondere einer Injektions- oder Infusionslösung, von Augen-, Nasen- und Ohrentropfen, eines Safts, einer Kapsel, einer Emulsion oder Suspension, eines Globuli, Styli, Aerosols, Puders, einer Paste, Creme oder Salbe eingesetzt.

Die erfindungsgemäßen Hemmstoffe von Faktor Xa oder die genannten Arzneimittel werden bvorzugt zur Diagnose, Therapie oder Prophylaxe einer kardiovaskulären Erkrankung oder eines thromboembolischen Ereignisses, insbesondere in oraler, subkutaner, intravenöser oder transdermaler Form verwendet.

Die Erfindung soll nachstehend anhand von drei Ausführungsbeispielen näher erläutert werden, ohne sie zu beschränken

### Methoden

Analytische HPLC: Shimadzu LC-10A System, Säule: Vydac C₁₈, 5 µm (250 x 4 mm) Lösungsmittel A: 0,1 % TFA in Wasser, B: 0,1 % TFA in ACN, Gradient: 10 % B bis 60 % B in 50 min, 1 ml/min Fluß, Detektion bei 220 oder 215 nm. Präparative HPLC: Shimadzu LC-8A System, Säule: Knauer C₁₈, 5 µm (250 x 32 mm) Lösungsmittel A: 0,1 % TFA in Wasser, B: 0,1 % TFA in ACN, Gradient: 10 % B bis 55 % B in 120 min, 10 ml/min Fluß, Detektion bei 220 nm. Massenspektroskopie: Die Massenspektren wurden auf einem Kompact Probe der Firma Kratos (Manchester, England) mit einem Flugzeitmessungsdetektor und α-Cyano-Hydroxyzimtsäure als Matrix, bzw. auf einem ESI-MS LCQ der Firma Finnigan (Bremen, Deutschland), gemessen.

### Beispiel 1: Synthese von Benzylsulfonyl-D-Ser(tBu)-Glu-4-Amidinobenzylamid x TFA (Reference)

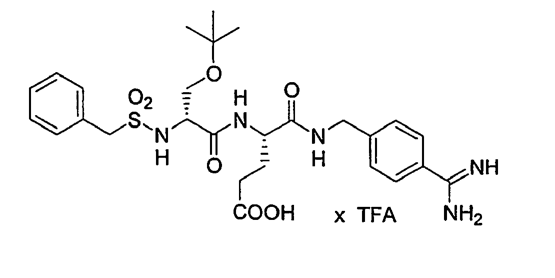

### 1.1 Boc-4-Cyanobenzylamid

20 g (0,151 mol) 4-Cyano-benzylamin wurden in 300 ml H₂O, 150 ml Dioxan und 150 ml 1 N NaOH gelöst. Unter Eiskühlung wurden 37,5 ml Di-tert.-butyl-dicarbonat zugetropft und eine Stunde bei 0 °C sowie weitere 24 Std. bei Raumtemperatur gerührt. Das Dioxan wurde im i.V. entfernt und das Produnkt wurde in Essigester und 5 % KHSO₄-Lösung aufgenommen. Die Essigesterphase wurde 3Mal mit 5 %-iger KHSO₄- und 3-mal mit gesättigter NaCl-Lösung gewaschen, über Na₂SO₄ getrocknet und i.V. eingeengt (weiße Kristalle). HPLC: Acetonitril/H₂O, Elution bei 44,1 % Acetonitril; Ausbeute: 30,48 g (0,131 mol), 87 %.

### 1.2 Boc-4-Acetyloxamidinobenzylamid

Nach Judkins et al. (Synthetic Comm. 26, 4351-4367, 1996) wurden 30,48 g (0,131 mol) Boc-4-Cyanobenzylamid mit 13,65 g (0,197 mol) Hydroxylamin x HCl und 34 ml (0,197 mol) DIEA in 300 ml abs. Ethanol gelöst. Es wurde 2 Std. unter Rückfluss gekocht und über Nacht bei Raumtemperatur gerührt. Danach wurde der Ansatz i.V. eingeengt, der Rückstand in ca. 200 ml Essigsäure gelöst und mit 18,67 ml (0,197 mol) Essigsäureanhydrid versetzt. Nach 1 Std. wurde erneut eingeengt, in Essigester gelöst und bei 0 °C je 3-mal mit 5 %iger KHSO₄-und gesättigter NaCl-Lösung gewaschen. Nach dem Trocknen über Na₂SO₄ und Einengen i.V. fiel ein weißes Pulver an. HPLC: Acetonitril/H₂O, Elution bei 32,0 % Acetonitril; Ausbeute: 31,3 g (0,102 mol) 78 %.

### 1.3 4-AcetyloxAmidinobenzylamin x HCl

5 mmol Boc-4-Acetyloxamidinobenzylamid werden in 20 ml 1 N HCl in Eisessig gelöst und 45 min bei Raumtemperatur stehen gelassen. Dann wird i.V. weitgehend eingeengt, das Produkt mit trockenem Diethylether gefällt, abgefrittet, nochmals mit frischem Ether gewaschen und i.V. getrocknet. Auf Grund der quantitativen Umsetzung wurde das Produkt ohne weitere Reinigung für den nächsten Syntheseschritt eingesetzt.

### 1.4 Boc-Glu(OBzl)-4-Acetyloxamidinobenzylamid

Die Kopplung von Boc-Glu(OBzl)-OH (Orpegen, Heidelberg) an 4-AcetyloxAmidinobenzylamin x HCl erfolgte nach Frérot et al. (Tetrahedron 47, 259 ff., 1991). Dazu wurden 2,27 g (9,3 mmol) 4-AcetyloxAmidinobenzylamin x HCl und 3,138 g g (9,3 mmol) Boe-Glu(OBzl)-OH in ca. 25 ml DMF gelöst. Bei 0 °C wurden 4,84 g (9,3 mmol) PyBOP und 3,878 ml (27,9 mmol) TEA zugegeben und der pH-Wert mit TEA auf 9 eingestellt. Nach 1 Std. Rühren bei Raumtemperatur wurde i.V. eingeengt, in Essigester aufgenommen und je 3-mal sauer, basisch und neutral gewaschen, mit Na₂SO₄ getrocknet und i.V. eingeengt. Ausbeute: 4,1 g (7,8 mmol) 84 %.

### 1.5 H-Glu(OBzl)-4-Acetyloxamidinobenzylamid x HCl

4,1 g (7,8 mmol) Boc-Glu(OBzl)-4-Acetyloxamidinobenzylamid wurden in 100 ml 1 N HCl in Eisessig gelöst und 45 min bei Raumtemperatur stehen gelassen. Dann wurde i.V. weitgehend eingeengt und mit trockenem Diethylether gefällt, danach abgefrittet und das Produkt nochmals mit frischem Ether gewaschen. Nach Trocknen des Produkts i.V. wurde es ohne weitere Aufreinigung für die Synthese nach Punkt 1.7 eingesetzt.

### 1.6 Benzylsulfonyl-D-Ser(tBu)-OH

525 mg (3,257 mmol) H-D-Ser(tBu)-OH und 1,187 ml (6,824 mmol) DIEA wurden in 75 ml 50 % Acetonitril gelöst. Dann wurden 591 mg (3,102 mmol) Benzylsulfonylchlorid zugegeben und 12 Std. bei Raumtemperatur gerührt. Es wurde i.V. eingeengt, mit Essigester aufgenommen und je 3-mal sauer und neutral gewaschen. Nach Trocknen über Natriumsulfat wurde i.V. eingeengt. Ausbeute: 743 mg (2,357mmol) 76 %.

### 1.7 Benzylsulfonyl-D-Ser(tBu)-Glu(OBzl)-4-Acetyloxamidinobenzylamid

136 mg (0,433 mmol) Benzylsulfonyl-D-Ser(tBu)-OH und 194 mg (0,433 mmol) H-Glu(OBzl)-4-Acetyloxamidinobenzylamid x HCl wurden in 5 ml abs. DMF gelöst. Unter Eiskühlung wurden 225 mg (0,433 mmol) PyBOP und 230 µl (1,32 mmol) DIEA zugegeben. Nach 2 Std. wurde i.V. eingeengt, mit Essigester aufgenommen und je 3-mal sauer, basisch und neutral gewaschen. Nach Trocknen über Natriumsulfat wurde i.V. eingeengt und ohne weitere Aufarbeitung nach Punkt 1.8 hydriert. Ausbeute: 242 mg (0,342 mmol) 79 %.

### 1.8 Benzylsulfonyl-D-Ser(tBu)-Glu-4-Amidinobenzylamid x TFA

242 mg (0,342 mmol) Bzls-D-Ser(tBu)-Glu(OBzl)-4-Acetyloxamidinobenzylamid wurden in 30 ml 90 %iger Essigsäure gelöst. Anschließend wurden unter Argon 20 mg 10 % Palladium auf Aktivkohle zugesetzt. Argon wurde durch eine Wasserstoffatmosphäre ersetzt und der Ansatz unter Rühren 24 Std. hydriert. Der Katalysator wurde abfiltriert, das Filtrat i.V. eingeengt und das Produkt mit präparativer reversed-phase HPLC gereinigt (Acetonitril/H₂O, 0,1 % Trifluoressigsäure, Elution bei 34,9 % Acetonitril).

### Beispiel 2: Hemmung von F Xa durch ausgewählte acylierte Amidinobenzylamin-Verbindungen (Reference)

| R₅ | Konfiguration R₄ | R₄ | R₃ | X-R₂ | Y-R₁ | Kᵢ(µM) |
|---|---|---|---|---|---|---|
| Bzl-SO₂ | D | CH₂-O-tBu | H | CH₂ | CH₂ | 0,050 |
| Bzl-SO₂ | D | CH₂-O-tBu | H | CH-CH₂-COOH | CH₂ | 1,2 |
| Bzl-SO₂ | D | CH₂-O-tBu | H | CH-(CH₂)₂-COOH | CH₂ | 0,25 |

### Bestimmung der Hemmwirkung

Zur Bestimmung der Hemmwirkung wurden 200 µI Tris-Puffer (0,05 M, 0,154 M NaCI, 5% Ethanol, pH 8,0; enthält den Inhibitor), 25 µI Substrat (Moc-D-Nle-Gly-Arg-pNA in H₂O; Pentapharm Ltd., Basel, Schweiz) und 50 µl F Xa (vom Rind, Diagnostic Reagents Ltd, Thame, GB) bei 25 °C inkubiert. Nach 3 min wurde die Reaktion durch Zugabe von 25 µI Essigsäure (50%) unterbrochen und die Absorption bei 405 nm mittels Microplate Reader (MR 5000, Dynatech, Denkendorf, Deutschland) bestimmt. Die Kᵢ-Werte wurden nach Dixon (Biochem. J. 55, 170-171, 1953) durch lineare Regression mittels eines Computerprogramms ermittelt. Die Kᵢ-Werte sind das Mittel aus mindestens drei Bestimmungen.

### Beispiel 3: Elimination nach i.v.-Gabe von 1 mg/kg Körpergewicht an der Ratte von Derivaten des Benzylsulfonyl-D-Ser(tBu)-Gly-4-amidinobenzylamids mit Ala, Asp bzw. Glu in P2-Position

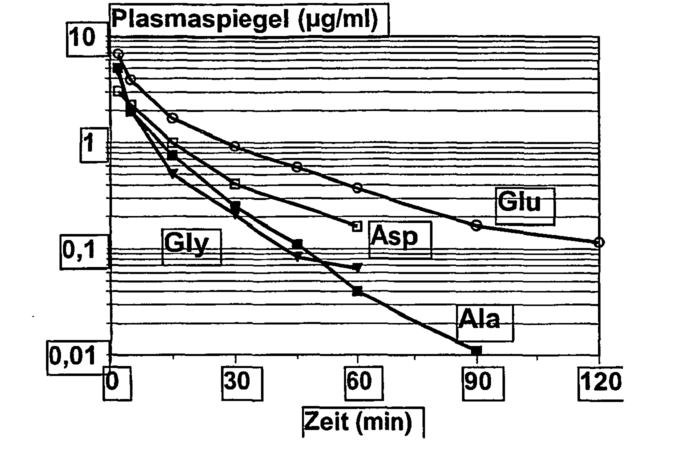

### Tierversuche

Weibliche Wistar Ratten (240-300 g Körpergewicht) wurden narkotisiert (Ethylurethan 2,5 g/ml in NaCl, 0,5 ml/100 g Ratte), anschließend erfolgte die Präparation der am Hals gelegenen *A. carotis.* Ein in diesem Gefäß fixierter Katheter ermöglichte die Blutentnahme zu festgelegten Zeiten. Das Applikationsvolumen betrug 0,5 ml, als Applikationslösung wurde 0,9% NaCl eingesetzt. Blutproben à 500 µl (versetzt im Verhältnis 19 + 1 mit 1,04 M Natriumcitrat) wurden zu folgenden Zeitpunkten entnommen: 2, 5, 15, 30, 45, 60, 90, 120, 150, 180, 210, 240 und 270 min. Der entstandene Blutverlust wurde unmittelbar nach Entnahme der Probe mit 500 µl 0,9 % NaCl-Lösung kompensiert. Citratplasma wurde durch Zentrifugation des Blutes bei 1200*g, für 10 min erhalten. Die Konzentration der Wirkstoffe im Plasma wurde mittels HPLC ermittelt.

### Beispiel 4: Hemmung von Faktor Xa durch Inhibitoren der allgemeinen Struktur nach Formel I

| Nr | R₅ | P₂ | P₁ | NH-YR₁-V-U-Z | Kᵢ (µM) |
|---|---|---|---|---|---|
| 1. | 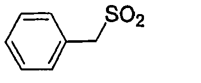 | D-Phe(3-Amidino) | Gly | 4-Amba | 0,0065 |
| 2. | 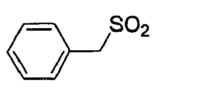 | D-Arg | Gly | 4-Amba | 0,0067 |
| 3. | 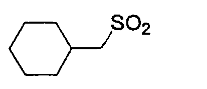 | D-Ser(tBu) | Gly | 4-Amba | 0,014 |
| 4. | 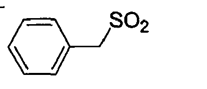 | D-Phe | Gly | 4-Amba | 0,026 |
| 5. | 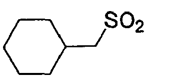 | D-Ser(tBu) | Ser | 4-Amba | 0,027 |
| 6. | 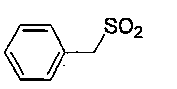 | D-Cha | Glu | 4-Amba | 0,028 |
| 7. | 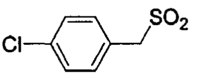 | D-Ser(tBu) | Gly | 4-Amba | 0,029 |
| 8. | 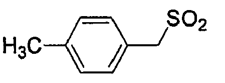 | D-Ser(tBu) | Gly | 4-Amba | 0,034 |
| 9. | 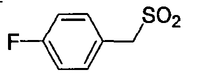 | D-Ser(tBu) | Gly | 4-Amba | 0,036 |
| 10. | 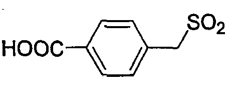 | D-Ser(tBu) | Gly | 4-Amba | 0,053 |
| 11. | 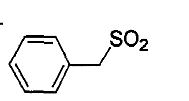 | D-Ser(tBu) | Ser | 4-Amba | 0,054 |
| 12. | 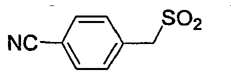 | D-Ser(tBu) | Gly | 4-Amba | 0,065 |
| 13. | 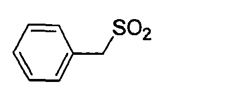 | D-Cha | Lys | 4-Amba | 0,067 |
| 14. | 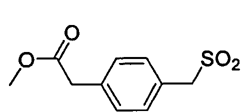 | D-Ser(tBu) | Gly | 4-Amba | 0,07 |
| 15. | 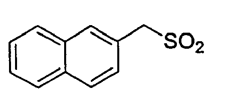 | D-Ser(tBu) | Gly | 4-Amba | 0,078 |
| 16. | 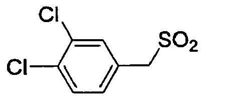 | D-Ser(tBu) | Gly | 4-Amba | 0,083 |
| 17. | 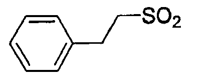 | D-Ser(tBu) | Gly | 4-Amba | 0,088 |
| 18. | 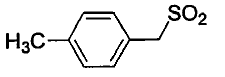 | D-Ser(tBu) | Ala | 4-Amba | 0,12 |
| 19. | 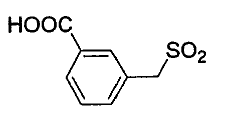 | D-Ser(tBu) | Gly | 4-Amba | 0,13 |
| 20. | 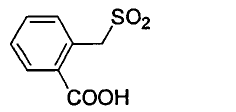 | D-Ser(tBu) | Gly | 4-Amba | 0,14 |
| 21. | 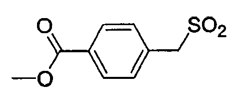 | D-Ser(tBu) | Gly | 4-Amba | 0,16 |
| 22. | 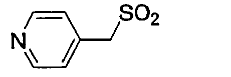 | D-Ser(tBu) | Gly | 4-Amba | 0,17 |
| 23. | 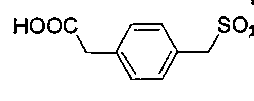 | D-Ser(tBu) | Gly | 4-Amba | 0,17 |
| 24. | 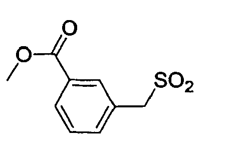 | D-Ser(tBu) | Gly | 4-Amba | 0,18 |
| 25. | 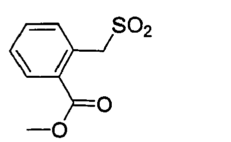 | D-Ser(tBu) | Gly | 4-Amba | 0,18 |
| 26. | 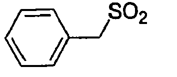 | D-Phe(4-Amidino) | Gly | 4-Amba | 0,26 |
| 27. | 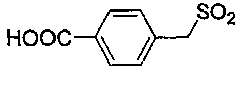 | D-Ser(tBu) | Ser | 4-Amba | 0,27 |
| 28. | 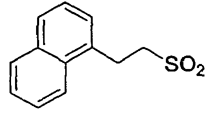 | D-Ser(tBu) | Gly | 4-Amba | 0,28 |
| 29. | 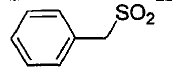 | D-Phe(4-CN) | Gly | 4-Amba | 0,30 |
| 30. | 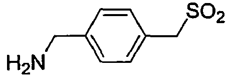 | D-Ser(tBu) | Gly | 4-Amba | 0,35 |
| 31. | 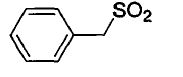 | D-Phe(4-Aminomethyl) | Gly | 4-Amba | 0,39 |
| 32. | 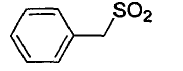 | D-His | Gly | 4-Amba | 0,67 |
| 33. | 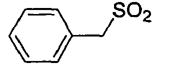 | D-Ser(tBu) | Gly | 4-Oxamidinobenzylamid | 26 |

### Beispiel 5: Synthese von Beispiel 1: 3-(HOOC)Benzylsulfonyl-dSer(tBu)-Gly-4-Amidinobenzylamid x TFA (Nr. 19. der Tabelle aus Beispiel 4)

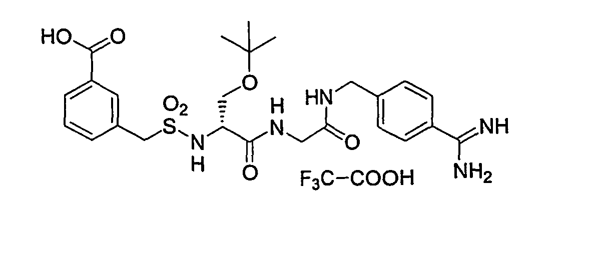

### 5a) 3-(COOMe)-Benzylsulfonsäure Natrium-Salz

5 g (21,1 mmol) 3-(Bromomethyl)Benzoesäuremethylester (Lancaster) wurden in 35 ml Wasser suspendiert und nach Zugabe von 2,94 g (23,3 mmol) Na₂SO₃ 8 h unter Rückfluss gekocht. Der Ansatz wurde heiß filtriert und das Wasser im Vakuum bis zur beginnenden Kristallisation eingeengt. Der Ansatz wurde über Nacht im Kühlschrank aufbewahrt, danach wurden die Kristalle abgesaugt und nochmals aus Wasser umkristallisiert. Die Kristalle wurden abgesaugt und im Vakuum getrocknet.
Ausbeute: 3,9 g (15,46 mmol) HPLC: 22,3 % B

### 5b) 3-(COOMe)-Benzylsulfonylchlorid

2,5 g (9,91 mmol) 3-(COOMe)-Benzylsulfonsäure Natrium-Salz wurden mit ca. 10 ml Phosphorylchlorid angefeuchtet, mit 2,27 g (10,9 mmol) PCl₅ versetzt und 15 Minuten im Eisbad gerührt. Danach wurde der Ansatz 4 h auf 80 °C erwärmt. Anschließend wurde der Ansatz auf Eis gegossen und 30 min kräftig gerührt, das Produkt schied sich in Form weißer Kristalle auf dem Eis ab. Nachdem das Eis teilweise aufgetaut war, wurde der Ansatz durch eine Fritte filtriert und das verbleibende Produkt-Eis-Gemisch mehrmals mit Wasser gewaschen. Die verbleibenden Kristalle wurden im Vakuum getrocknet.
Ausbeute: 1,6 g (6,43 mmol) 65 % (weiße Kristalle)

### 5c) 3-(COOMe)-Benzylsulfonyl-dSer(tBu)-OH

0,75 g (4,65 mmol) H-dSer(tBu)-OH (Bachem) wurden in 60 ml trockenem DCM suspendiert, mit 1,23 ml (9,765 mmol) Trimethylsilylchlorid und 1,7 ml (9,765 mmol) DIEA versetzt. Der Ansatz wurde 1,0 h unter Rückfluss gekocht und danach im Eisbad abgekühlt. Anschließend wurden 1,27 g (5,12 mmol) 3-(COOMe)-Benzylsulfonylchlorid und 1,04 ml (6 mmol) DIEA in mehreren Portion innerhalb von 30 min zugegeben. Der Ansatz wurde weitere 15 min unter Eiskühlung und danach für 3 h bei Raumtemperatur gerührt. Das Lösungsmittel wurde im Vakuum entfernt, der Rückstand in Wasser (mit 1 N NaOH auf pH 8,5-9 gebracht) gelöst und 2 x mit Ether extrahiert. Die wässrige Phase wurde mit 5 % KHSO₄-Lösung angesäuert und 3 x mit Essigester extrahiert. Die vereinte Essigesterphase wurde jeweils 3 x mit 5 % KHSO₄-Lösung und NaCl-gesättigter Lösung gewaschen und mit Na₂SO₄ getrocknet. Anschließend wurde das Lösungsmittel im Vakuum entfernt.
Ausbeute: 1,3 g (3,48 mmol Feststoff), HPLC: 51 % B

### 5d) H-Gly-4-(Acetyloxamidino)Benzylamid x HCl

2 g (5,49 mmol) Boc-Gly-4-(Acetyloxamidino)Benzylamid (hergestellt wie in WO 01/96286 A2 beschrieben) wurden mit 30 ml 1 N HCl in Eisessig versetzt. Der Ansatz wurde gelegentlich umgeschüttelt. Nach 45 min wurde das Lösungsmittel etwas eingeengt und das Produkt durch Zugabe von Diethylether gefällt, auf einer Fritte abgesaugt, mit Ether gewaschen und im Vakuum getrocknet.
Ausbeute: 1,55 g (5,15 mmol), weißer Feststoff

### 5e) 3-(COOMe)-Benzylsulfonyl-dSer(tBu)-Gly-4-(Acetyloxamidino)Benzylamid

1 g (2,68 mmol) 3-(COOMe)-Benzylsulfonyl-dSer(tBu)-OH und 0,84 g (2,8 mmol) H-Gly-4-(Acetyloxamidino)Benzylamid x HCl wurden unter Rühren und Eiskühlung in 15 ml DMF gelöst und mit 1,39 g (2,68 mmol) PyBop sowie 1,26 ml (7,236 mmol) DIEA versetzt. Nach 30 min wurde das Eisbad entfernt und weitere 4 h bei Raumtemperatur gerührt. Das DMF wurde im Vakuum eingeengt, der verbleibende Rückstand in Essigester gelöst und jeweils 3 x mit 5 % KHSO₄, NaCl-gesättigtem Wasser, gesättigter NaHCO₃-Lösung und nochmals mit NaCl-gesättigtem Wasser gewaschen. Die Essigesterphase wurde mit Na₂SO₄ getrocknet und anschließend das Lösungsmittel im Vakuum entfernt. Das Rohprodukt wurde ohne weitere Reinigung für den nächsten Syntheseschritt verwendet.
Ausbeute: 1,35 g (2,18 mmol) Öl, HPLC: 47,89 % B

### 5f) 3-(COOMe)-Benzylsulfonyl-dSer(tBu)-Gly-4-(Amidino)Benzylamid x Acetat

1 g (1,61 mmol) 3-(COOMe)-Benzylsulfonyl-dSer(tBu)-Gly-4-(Acetyloxamidino)-Benzylamid wurden in 65 ml 90 % Essigsäure gelöst, mit 150 mg Katalysator (10 % Pd auf Aktivkohle) versetzt und über Nacht mit Wasserstoff hydriert. Der Katalysator wurde abfiltriert und das Lösungsmittel im Vakuum eingeengt. Der verbleibende Rückstand wurde mit Toluen versetzt, danach wurde das Lösungsmittel im Vakuum wieder entfernt. Dieser Vorgang wurde nochmals wiederholt. Der verbleibende Rückstand wurde direkt für den nächsten Reaktionsschritt verwendet.
Ausbeute: 0,9 g (1,44 mmol) Feststoff, HPLC: 39,75 % B
Ca. 50 mg des Rohproduktes wurden mit präparativer reversed-phase HPLC gereinigt und lyophilisiert.
MS: berechnet 561,2 (monoisotopic), gefunden 562,9 [M+H]⁺

### 5g) 3-(COOH)-Benzylsulfonyl-dSer(tBu)-Gly-4-(Amidino)Benzylamid x TFA

750 mg (1,2 mmol) 3-(COOMe)-Benzylsulfonyl-dSer(tBu)-Gly-4-(Amidino)-Benzylamid x Acetat wurden in 20 ml Methanol und 10 ml Wasser gelöst und mit 4 ml 1 N LiOH versetzt. Der Ansatz wurde über Nacht gerührt, nach ca. 15 h mit 5 % KHSO₄ neutralisiert (pH 6-7) und das Lösungsmittel im Vakuum entfernt. Das Rohprodukt wurde mittels präparativer reversed-phase HPLC gereinigt und lyophilisiert.
HPLC: 34,16 % B (weißer Feststoff)
MS: berechnet 547,21 (monoisotopic), gefunden 548,3 [M+H]⁺

### Beispiel 6: Benzylsulfonyl-dSer-Ser-4-Amidinobenzylamid x TFA (Nr. 11 der Tabelle aus Beispiel 4) (Reference)

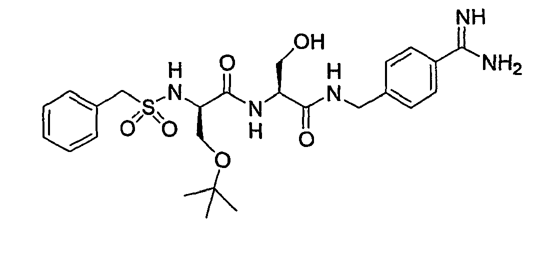

### 6a) Boc-Ser-4-(Acetyloxamidino)Benzylamid

1 g (4,873 mmol) Boc-Ser-OH wurden in 50 ml DMF gelöst und bei -15 °C mit 0,536 ml (4,873 mmol) NMM und 0,6335 ml (4,873 mmol) CKBIE versetzt. Der Ansatz wurde 10 min bei -15 °C gerührt, dann wurden 1,187 g (4,873 mmol) 4-(Acetyloxamidino)Benzylamin x HCl (hergestellt wie in WO 01/96286 A2 beschrieben) und 0,536 ml (4,873 mmol) NMM hinzugefügt. Nach 20 min wurden nochmals 0,15 ml NMM zum Ansatz gegeben. Der Ansatz wurde eine weitere Stunde bei -15°C und über Nacht bei Raumtemperatur gerührt. Das Lösungsmittel wurde im Vakuum entfernt, der Ansatz in reichlich Essigester aufgenommen und 1 x mit wenig ges. NaHCO₃-Lösung und 2 x mit wenig NaCl-gesättigtem Wasser gewaschen und mit Na₂SO₄ getrocknet. Das Lösungsmittel wurde im Vakuum entfernt.
Ausbeute: 1,2 g weißer Schaum, HPLC: 29,9 % B

### 6b) H-Ser-4-(Acetyloxamidino)Benzylamid x TFA

1,1 g Boc-Ser-4-(Acetyloxamidino)Benzylamid wurden mit 2 ml Wasser und 18 ml TFA versetzt. Nach 1 h Rühren bei Raumtemperatur wurde das Produkt durch Zugabe von trockenem Diethylether gefällt, abgesaugt und nochmals mit Diethylether gewaschen. Das Produkt wurde im Vakuum getrocknet.
Ausbeute: 0,85 g weißer Feststoff, HPLC: 15,42 % B

### 6c) Bzls-dSer(tBu)-Ser-4-(Acetyloxamidino)Benzylamid

0,2 g (0,634 mmol) Bzls-dSer(tBu)-OH und 0,2097 g (0,634 mmol) H-Ser-4-(Acetyloxamidino)Benzylamid x TFA wurden in 10 ml DMF gelöst und bei 0 °C mit 0,329 g (0,634 mmol) PyBop und 329 µl DIEA versetzt. Der Ansatz wurde 30 min bei 0 °C und weitere 4 h bei Raumtemperatur gerührt. Das Lösungsmittel wurde im Vakuum entfernt, der Rückstand in reichlich Essigester aufgenommen und jeweils 2 x mit wenig Volumen an 5 % KHSO₄, NaCl-gesättigtem Wasser, gesättigter NaHCO₃-Lösung und nochmals mit NaCl-gesättigtem Wasser gewaschen und anschließend mit Na₂SO₄ getrocknet. Das Lösungsmittel wurde im Vakuum entfernt. Es verblieb ein öliges Rohprodukt, das direkt für den nächsten Syntheseschritt eingesetzt wurde.
Ausbeute: 0,31 g Öl, HPLC: 42,97 % B

### 6d) Bzls-dSer(tBu)-Ser-4-(Amidino)Benzylamid x TFA

200 mg (0,29 mmol) Bzls-dSer(tBu)-Ser(Bzl)-4-(Acetyloxamidino)Benzylamid wurden in 100 ml 90 % Essigsäure gelöst, dazu wurden 50 mg Katalysator (10 % Pd/C) gegeben. Der Ansatz wurde 6 h bei Raumtemperatur und Normaldruck mit Wasserstoff hydriert. Anschließend wurde der Katalysator abfiltriert, das Lösungsmittel im Vakuum eingeengt und das Produkt mit präparativer HPLC gereinigt und lyophilisiert.
Ausbeute: 75 mg (weisser Feststoff); HPLC: 34,7 % B.
MS: berechnet 533,23 (monoisotopic), gefunden 534,5 [M+H]⁺

### Beispiel 7: 4-(Aminomethyl)Benzylsulfonyl-dSer(tBu)-Gly-4-Amidinobenzylamid x 2 TFA (Reference)

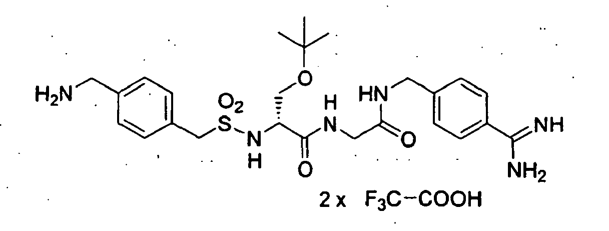

### 7a) 4-Cyano-Benzylsulfonsäure Natrium-Salz

30 g (153 mmol) 4-Cyanobenzylbromid (Aldrich) wurden in 150 ml Wasser suspendiert und nach Zugabe von 21,2 g (168,3 mmol) Na₂SO₃ 8 h unter Rückfluß gekocht. Der Ansatz wurde heiß filtriert und das Wasser im Vakuum etwas eingeengt. Der Ansatz wurde zur Kristallisation über Nacht im Kühlschrank aufbewahrt, danach wurden die Kristalle abgesaugt und nochmals aus Wasser umkristallisiert. Die Kristalle wurden abgesaugt und im Vakuum getrocknet.
Ausbeute: 17,1 g (78 mmol),HPLC: 18,24 % B

### 7b) 4-Cyano-Benzylsulfonylchlorid

5 g (22,83 mmol) 4-Cyano-Benzylsulfonsäure Natrium-Salz wurden mit ca. 20 ml Phosphorylchlorid angefeuchtet und mit 5,2 g (25,11 mmol) PCl₅ versetzt und 15 min unter Eiskühlung gerührt. Anschließend wurde der Ansatz 4 h auf 80°C erwärmt. Danach wurde der Ansatz auf Eis gegossen und 30 min kräftig gerührt, das Produkt schied sich als weißer Feststoff auf dem Eis ab. Nachdem das Eis teilweise aufgetaut war, wurde der Ansatz durch eine Fritte filtriert und das verbleibende Produkt-Eis-Gemisch mehrmals mit Wasser gewaschen. Die verbleibenden Kristalle wurden im Vakuum getrocknet und direkt für den nächsten Syntheseschritt verwendet.
Ausbeute: 3,4 g (15,76 mmol)

### 7c) 4-Cyano-Benzylsulfonyl-dSer(tBu)-OH

1 g (6,2 mmol) H-dSer(tBu)-OH (Bachem) wurden in 50 ml trockenem DCM suspendiert, mit 1,65 ml (13 mmol) Trimethylsilylchlorid und 2,26 ml (13 mmol) DIEA versetzt. Der Ansatz wurde 1 h unter Rückfluß gekocht und im Eisbad abgekühlt. Anschließend wurden 1,47 g (6,82 mmol) 4-Cyano-Benzylsulfonylchlorid und 1,19 ml (6,82 mmol) DIEA innerhalb von 30 min zugesetzt. Der Ansatz wurde weitere 15 min unter Eiskühlung und danach für weitere 3 h bei Raumtemperatur gerührt. Das Lösungsmittel wurde im Vakuum entfernt, der Rückstand in Wasser (mit 1 N NaOH auf pH 8,5-9 gebracht) gelöst und 2 x mit Ether extrahiert. Anschließend wurde die wässrige Phase mit 5 % KHSO₄-Lösung angesäuert und 3 x mit Essigester extrahiert. Die vereinte Essigesterphase wurde jeweils 3 x mit 5 % KHSO₄-Lösung und NaCl-gesättigter Lösung gewaschen und mit Na₂SO₄ getrocknet. Das Lösungsmittel wurde im Vakuum entfernt. Ausbeute: 1,4 g (4,11 mmol Feststoff), HPLC: 48,89 % B

### 7d) 4-Cyano-Benzylsulfonyl-dSer(tBu)-Gly-4-(Acetyloxamidino)Benzylamid

1 g (2,94 mmol) 4-Cyano-Benzylsulfonyl-dSer(tBu)-OH und 0,884 g (2,94 mmol) H-Gly-4-(Acetyloxamidino)Benzylamid x HCl (siehe Beispiel 1d) wurden unter Rühren und Eiskühlung in 15 ml DMF gelöst und mit 1,53 g (2,94 mmol) PyBop sowie 1,38 ml (7,94 mmol) DIEA versetzt. Nach 30 min wurde das Eisbad entfernt und der Ansatz weitere 4 h bei Raumtemperatur gerührt. Das DMF wurde im Vakuum eingeengt, der verbleibende Rückstand in Essigester gelöst und jeweils 3 x mit 5 % KHSO₄, NaCl-gesättigtem Wasser, gesättigter NaHCO₃-Lösung und nochmals mit NaCl-gesättigtem Wasser gewaschen und mit Na₂SO₄ getrocknet. Das Lösungsmittel wurde im Vakuum entfernt. Das Rohprodukt wurde ohne weitere Reinigung für den nächsten Syntheseschritt verwendet.
Ausbeute: 1,4 g (2,386 mmol) Öl, HPLC: 46,05 % B

### 7e) 4-Cyano-Benzylsulfonyl-dSer(tBu)-Gly-4-(Amidino)Benzylamid x Acetat

1 g (1,7 mmol) 4-Cyano-Benzylsulfonyl-dSer(tBu)-Gly-4-(Acetyloxamidino)-Benzylamid wurden in 70 ml 90 % Essigsäure gelöst, mit 150 mg Katalysator (10 % Pd auf Aktivkohle) versetzt und 5 h mit Wasserstoff hydriert. Der Katalysator wurde abfiltriert und das Lösungsmittel eingeengt. Der verbleibende Rückstand wurde mit Toluen versetzt, anschließend wurde das Lösungsmittel im Vakuum entfernt. Dieser Vorgang wurde nochmals wiederholt. Der verbleibende Rückstand wurde direkt für den nächsten Reaktionsschritt verwendet.
Ausbeute: 0,85 g (1,44 mmol als Acetat-Salz) Feststoff HPLC: 37,55 % B
Ca. 60 mg dieses Rohproduktes wurden mit präparativer HPLC gereinigt.
MS: berechnet 528,2 (monoisotopic), gefunden 530,1 [M+H]⁺

### 7f) 4-Aminomethyl-Benzylsulfonyl-dSer(tBu)-Gly-4-(Amidino)Benzylamid x 2 TFA

200 mg Rohprodukt an 4-Cyano-Benzylsulfonyl-dSer(tBu)-Gly-4-(Amidino)-Benzylamid x Acetat wurden in 50 ml 90 % Essigsäure und 5 ml 1 N HCl gelöst, mit 40 mg Katalysator (10 % Pd auf Aktivkohle) versetzt und über Nacht bei 40 °C mit Wasserstoff hydriert. Der Katalysator wurde abfiltriert und das Lösungsmittel im Vakuum eingeengt. Der verbleibende Rückstand wurde mit präparativer reversed phase HPLC gereinigt.
Ausbeute: 75 mg (als 2 x TFA-Salz) Feststoff HPLC: 26,05 % B
MS: berechnet 532,25 (monoisotopic), gefunden 533,7 [M+H]⁺

### Beispiel 8: Benzylsulfonyl-dPhe(3-Amidino)-Gly-4-(Amidino)Benzylamid x2TFA

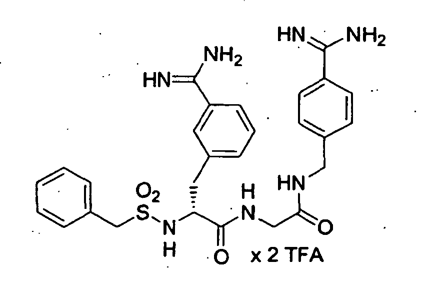

### 8a) Benzylsulfonyl-dPhe(3-CN)-OH

1 g (4,42 mmol) H-dPhe(3-CN)-OH x HCl wurden in einer Mischung von 40 ml Dioxan und 10 ml Wasser gelöst, der pH wurde durch Zugabe von DIEA auf pH 8-9 eingestellt. Der Ansatz wurde im Eisbad gekühlt, über einen Zeitraum von 3 h wurden insgesamt 1,265 g (6,63 mmol) in mehreren Portionen zugegeben, wobei der pH-Wert durch Zugabe von DIEA auf 8-9 eingestellt wurde. Nach 1 h wurde das Eisbad entfernt und der Ansatz über Nacht bei RT gerührt. Das Lösungsmittel wurde im Vakuum entfernt, der Rückstand in Essigester und 5 % KHSO₄-Lösung aufgenommen. Die Essigesterphase wurde 3 x mit 5 % KHSO₄-Lösung und 3 x mit NaCl-gesättigter wässriger Lösung gewaschen. Die Essigesterphase wurde mit Na₂SO₄ getrocknet und das Lösungsmittel im Vakuum entfernt. Das Produkt wurde aus Essigester kristallisiert.
Ausbeute: 1,6 g (weißer Feststoff), HPLC bei 45,8 % B

### 8b) Benzylsulfonyl-dPhe(3-Acetyloxamidino)-OH

800 mg (2,32 mmol) Benzylsultonyl-dPhe(3-CN)-OH wurden in 30 ml Methanol gelöst und mit 280 mg (4 mmol) Hydroxylamin x HCl und 0,63 ml (3,6 mmol) DIEA versetzt. Der Ansatz wurde 5 h unter Rückfluß gekocht und über Nacht bei RT gerührt. Das Lösungsmittel wurde im Vakuum entfernt und der Rückstand in 30 ml Essigsäure gelöst. Es wurden 665 µl (7 mmol) Essigsäureanhydrid zugegeben, der Ansatz wurde 30 min bei RT gerührt. Danach wurde das Lösungsmittel im Vakuum entfernt und der Rückstand in Essigester gelöst und 2 x mit 5 % KHSO₄-Lösung und 3 x mit NaCl-gesättigter wässriger Lösung gewaschen. Die Essigesterphase wurde mit Na₂SO₄ getrocknet und das Lösungsmittel im Vakuum entfernt. Ausbeute: 805 mg (Öl), HPLC bei 38,5 % B

### 8c) Benzylsulfonyl-dPhe(3-Acetyloxamidino)-Gly-4-(Acetyloxamidino) Benzylamid

100 mg (0,24 mmol) Benzylsulfonyl-dPhe(3-Acetyloxamidino)-OH und 75 mg (0,25 mmol) H-Gly-4-(Acetyloxamidino)Benzylamid x HCl (siehe Beispiel 1d) wurden unter Rühren und Eiskühlung in 5 ml DMF gelöst und mit 125 mg (0,24 mmol) PyBop sowie 125 µl DIEA versetzt. Nach 30 min wurde das Eisbad entfernt und der Ansatz weitere 3 h bei Raumtemperatur gerührt. Das DMF wurde im Vakuum eingeengt, der verbleibende Rückstand in Essigester gelöst und jeweils 3 x mit 5 % KHS04, NaCl-gesättigtem Wasser, gesättigter NaHCO₃-Lösung und nochmals mit NaCl-gesättigtem Wasser gewaschen und mit Na₂SO₄ getrocknet. Das Lösungsmittel wurde im Vakuum entfernt. Das Rohprodukt wurde ohne weitere Reinigung für den nächsten Syntheseschritt verwendet.
Ausbeute: 1,43 mg gelbliches Öl, HPLC: 38,3 % B

### 8d) Benzylsulfonyl-dPhe(3-Amidino)-Gly-4-(Amidino)Benzylamid

100 mg Benzylsulfonyl-dPhe(3-Acetyloxamidino)-Gly-4-(Acetyloxamidino) Benzylamid wurden in 20 ml Eisessig gelöst und mit 20 mg Katalysator (10 % Pd/C) versetzt. Der Ansatz wurde über Nacht unter Wasserstoffatmosphäre hydriert. Der Katalysator wurde abfiltriert und das Lösungsmittel im Vakuum entfernt. Das Produkt wurde mit präparativer HPLC gereinigt.
Ausbeute: 25 mg, HPLC bei 24,6 % B
MS: berechnet 549,22 (monoisotopic), gefunden 550,3 [M+H]⁺

### Verwendete Abkürzungen:

- Ac: Acetyl
- 4-Amba: 4-Amidinobenzylamid
- Boc: tert.-Butyloxycarbonyl
- Bzl: Benzyl
- dCha: d-βCyclohexylalanin
- CKIBE: Chlorkohlensäureisobutylester
- Dab: α,γ-Diaminobuttersäure
- Dap: α,β-Diaminopropionsäure
- DIEA: Diisopropylethylamin
- DMF: N,N-Dimethylformamid
- i.V.: im Vakuum
- NMM: N-Methylmorpholin
- PyBOP: Benzotriazol-1-yl-oxy-tris(pyrrolidino)phosphoniumhexafluorophosphat
- TEA: Triethylamin
- TFA: Trifluoressigsäure
- THF: Tetrahydrofuran
- tBu: tert.-Butyl

## Patentansprüche

1. Verbindung der allgemeinen Formel I 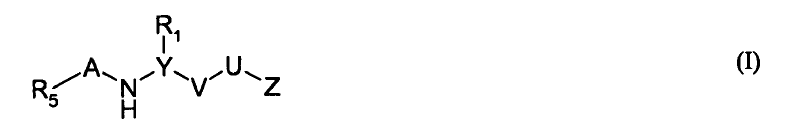 wobei
A P₂-P₁ mit 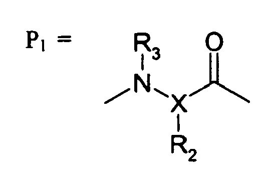 und
P₂ von einer der folgenden Aminosäuren in der D-Konfiguration abstammt: D-2,3-Diaminopropionsäure, D-2,4-Diaminobuttersäure, D-Citrullin, D-Homocitrullin, D-Norcitrullin, D-Arginin, D-Homoarginin, D-Norarginin, D-3-Guanidinophenylalanin, D-3-Guanidinophenylhomoalanin, D-3-Guanidinophenylglycin, D-3-Amidinophenylalanin, D-3-Amidinophenylhomoalanin, D-3-Amidinophenylglycin, D-4-Guanidinomethylphenylalanin, D-4-Guanidinomethylphenylhomoalanin, D-4-Guanidinomethylphenylglycin, D-3-Guanidinomethylphenylalanin, D-3-Guanidinomethylphenylhomoalanin, D-3-Guanidinomethylphenylglycin, D-3-Amidinopiperidinylalanin, D-3-Amidinopiperidinylhomoalanin, D-3-Amidinopiperidinylglycin, n-Butylamidinoglycin, n-Pentylamidinoglycin, n-Propylamidinoglycin, D-Canavanin, D-Homocanavanin, D-Norcanavanin, 2-Amino-4-Amidinohydrazono-Buttersäure, 2-Amino-3-Amidinohydrazono-Propionsäure, 2-Amino-5-Amidinohydrazono-Pentansäure, 2-Amino-4-(Pyridin-4-ylamino)-Buttersäure, 2-Amino-4-(Pyridin-4-ylamino)-Propionsäure, 2-Amino-4-(Pyridin-4-ylamino)-Pentansäure, 4-Imidazolyl-Propargylglycin, D-Histidin, D-Homohistidin, D-Histidin-(1-Methyl), D-Homohistidin-(1-Methyl), D-Histidin-(3-Methyl), D-Homohistidin-(3-Methyl), D-Alanin(4-[5-2(-amino)imidazoyl], D-Homoalanin(4-[5-2(-amino)imidazoyl], D-Glycin(4-[5-2(-amino)imidazoyl], D-Alanin(3-(2-Pyrimidinyl), D-Homoalanin(3-(2-Pyrimidinyl), D-Alanin(3-(5-Pyrimidinyl), D-Homoalanin(3-(5-Pyrimidinyl), D-2-Amino-3-(2-amino-pyrimidin-5-yl)-propionsäure, D-2-Amino-4-(2-amino-pyrimidin-5-yl)-buttersäure, D-Alanin(3-(2-benzimidazolyl)), D-Homoalanin(3-(2-benzimidazolyl)), D-Tryptophan das mit Aminoalkylgruppen am Indolring substituiert ist, D-Homotryptophan das mit Aminoalkylgruppen am Indolring substituiert ist, D-2-Amino-3-(6-amino-pyridin-3-yl)-propionsäure, D-2-Amino-4-(6-amino-pyridin-3-yl)-buttersäure, D-2-Amino-3-(6-amino-2-methylpyridin-3-yl)-propionsäure, D-2-Amino-4-(6-amino-2-methyl-pyridin-3-yl)-buttersäure, D-2-Amino-3-(6-amino-2,4-dimethyl-pyridin-3-yl)-propionsäure, D-2-Amino-4-(6-amino-2,4-dimethyl-pyridin-3-yl)-buttersäure, D-3-Hydroxyamidinophenylalanin, D-3-Hydroxy-amidinophenylhomoalanin, D-3-Hydroxyamidinophenylglycin,;
R₁ H oder -(CH₂)ₐCOOR₆ mit a = 0, 1, 2, 3, 4 oder 5 ist, wobei R₆ ein verzweigter oder unverzweigter Alkylrest mit 1 bis 6 C-Atomen ist;
R₂ ein H, ein verzweigter oder unverzweigter Alkylrest mit 1 bis 8 C-Atomen, oder
-(CH₂)_{c}COOR₈ mit c = 1, 2, 3 oder 4, wobei R₈ H oder ein verzweigter oder unverzweigter Alkylrest mit 1 bis 6 C-Atomen ist, oder
-(CH₂)_{d}-OR₉ mit d = 1, 2, 3 oder 4, wobei R₉ H ist, oder
-(CH₂)ₑOR₁₀, -(CH₂)ₑSR₁₀, -(CH₂)ₑ-Guanidino, -(CH₂)ₑ-Imidazol oder
-(CH₂)ₑNHR₁₀ mit e = 1, 2, 3, 4 oder 5 ist, wobei R₁₀ H, ein verzweigter oder unverzweigter Alkylrest mit 1-16 C-Atomen oder ein substituierter oder unsubstituierter Aryl-, Heteroaryl-, Aralkyl- oder Heteroaralkylrest
ist, wobei der Alkylrest 1 bis 16 C-Atome, und der Aryl- oder Heteroarylrest 4 bis 14 C-Atome besitzt;
R₃ H oder -(CH₂)_{b}R₇ mit b = 1, 2, 3, 4, 5, 6, 7 oder 8 ist, wobei R₇ H, ein verzweigter oder unverzweigter Alkylrest mit 1 bis 10 C-Atomen oder ein geladener Rest ist;
R₅ -(CH₂)g(CH₃)ₕ, -(CH₂)ᵢ-Aryl mit g + h = i = 0, 1, 2 oder 3, -SO₂R₁₂, -COR₁₂, oder -COOR₁₂ ist, wobei R₁₂ ein verzweigtes oder unverzweigtes Alkyl mit 1-16 C-Atomen, ein substituierter oder unsubstituierter Aryl-, Heteroaryl-, Aralkyl- oder Heteroaralkylrest, ein Adamantyl-, ein Campher-, ein Cyclohexylmethylrest ist,
wobei R₅ mit einer geladenen oder ungeladenen Gruppe ist;
U ein Phenyl- oder Cyclohexylrest ist;
ein Heterophenyl- oder Heterocyclohexylrest ist oder
ein Thiophenrest ist;
V (CH₂)ₙ mit n = 0, 1, 2 oder 3 ist;
X N oder CH ist;
Y N oder (CH)ₘ mit m = 0 oder 1 ist;
Z in 3- oder 4-Position vorkommt und eine Aminomethyl-, eine Guanidinofunktion oder eine Amidinogruppe 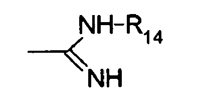 ist, wobei R₁₄ H, OH, NH₂, -COR₁₅ oder -COOR₁₅ ist, wobei R₁₅ ein verzweigter oder unverzweigter Alkylrest mit 1 bis 16 C-Atomen oder ein substituierter oder unsubstituierter Aryl- oder Heteroaryl-, Aralkyl- oder Heteroaralkylrest ist, wobei der Alkylrest vorzugsweise 1 bis 16 C-Atome und der Aryl- oder Heteroarylrest vorzugsweise 4 bis 14 C-Atome besitzt;
oder eine Verbindung mit
R₅ gleich 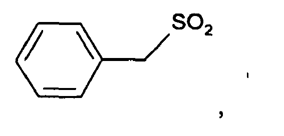 P₁ gleich 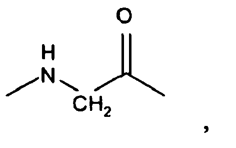 NH-YR₁-V-U-Z gleich 4-Amidinobenzylamid und P₂ ausgewählt aus D-Phe(3-Amidino) oder D-Arg;
**dadurch gekennzeichnet, dass** ein oder mehrere geladene Reste in den Resten R₁, R₂, R₃ oder R₅ vorhanden sind.

2. Verbindung nach Anspruch 1, wobei U an 1, 2 oder 3 Positionen vorzugsweise mit einem Halogen, insbesondere Fluor oder Chlor, oder einem Methyl, Ethyl-, Propyl-, Methoxy-, Ethoxy-, oder Propoxyrest substituiert ist.

3. Verbindung nach Anspruch 1 oder 2, wobei eine Carboxylgruppe als Ester, bevorzugt als Ethylester, geschützt vorliegt.

4. Verbindung nach mindestens einem der Ansprüche 1 bis 3, wobei R₉ in diesem Fall ein Alkylcarbonyl-, Aralkylcarbonyl-, Alkyloxycarbonyl- oder Aralkyloxycarbonyl-Rest ist, wobei der Alkylrest vorzugsweise 1 bis 6, insbesondere 1 bis 4 C-Atome und der Arylrest vorzugsweise 5 bis 8, insbesondere 6 C-Atome besitzt.

5. Verbindung der allgemeinen Formel I, **dadurch gekennzeichnet, dass** die Verbindung die folgende Struktur aufweist:

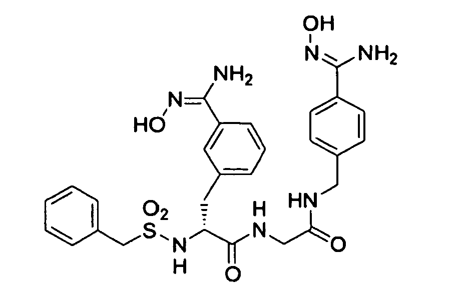

6. Verbindung nach mindestens einem der Ansprüche 1 bis 5, **dadurch gekennzeichnet, dass** der Substituent am substituierten Aryl-, Heteroaryl-, Aralkyl- oder Heteroaralkylrest ein Halogen, vorzugsweise Fluor, Chlor oder Brom, insbesondere Fluor oder Chlor, ist.

7. Verbindung nach mindestens einem der Ansprüche 1 bis 6, **dadurch gekennzeichnet, dass** die Verbindungen als Salze mit Mineralsäuren, bevorzugt als Hydrochloride, oder als Salze mit geeigneten organischen Säuren vorliegen.

8. Verbindung nach Anspruch 7, **dadurch gekennzeichnet, dass** Salze von Mineralsäuren auch Sulfate sind und geeignete organische Säuren Essigsäure, Ameisensäure, Methylsulfonsäure, Bernsteinsäure, Apfelsäure oder Trifluoressigsäure sind, wobei bevorzugte Salze von organischen Säuren Acetate sind.

9. Verbindung gemäß mindestens einem der Ansprüche 1 bis 8, **dadurch gekennzeichnet, dass** R₃ ein geladener Rest mit einer (CH₂)ⱼCOOR₁₃, -(CH₂)ⱼSO₂R₁₃, -(CH₂)ⱼNH₂, -(CH₂)ⱼ-Amidino-, -(CH₂)ⱼ-Hydroxyamidino- oder -(CH₂)ⱼ-Guanidino-Gruppe mit j = 0, 1 oder 2 ist, wobei R₁₃ H oder ein Alkylrest mit vorzugsweise 1 bis 6 C-Atomen, insbesondere 1 bis 4, vor allem Ethyl, ist.

10. Verbindung gemäß mindestens einem der Ansprüche 1 bis 9, **dadurch gekennzeichnet, dass** R₅ mit einem geladenen Rest ausgewählt aus einer -(CH₂)ⱼCOOR₁₃, -(CH₂)ⱼSO₂R₁₃, -(CH₂)ⱼNH₂, -(CH₂)ⱼ-Amidino-, -(CH₂)ⱼ-Hydroxyamidino- oder -(CH₂)ⱼ-Guanidino-Gruppe mit j = 0, 1 oder 2 modifiziert ist, wobei R₁₃ H oder ein Alkylrest mit vorzugsweise 1 bis 6 C-Atomen, insbesondere 1 bis 4, vor allem Ethyl, ist.

11. Verbindung ausgewählt aus
Benzylsulfonyl-dPhe(3-Amidino)-Gly-4-(Amidino)Benzylamid oder
Benzylsulfonyl-dArg-Gly-4-(Amidino)Benzylamid.

12. Verfahren zur Herstellung einer Verbindung gemäß mindestens einem der Ansprüche 1 bis 11, **dadurch gekennzeichnet, dass** sequentiell die entsprechenden Aminosäuren an ein 4-Acetyloxamidinobenzylamin angekoppelt werden, wobei die N-terminale Aminosäure entweder den R₅-Rest bereits trägt oder dieser anschließend daran gebunden wird.

13. Arzneimittel enthaltend eine Verbindung gemäß mindestens einem der Ansprüche 1 bis 11 sowie pharmazeutisch geeignete Hilfs- und/oder Zusatzstoffe.

14. Arzneimittel nach Anspruch 13, wobei das Arzneimittel in Form einer Tablette, eines Dragees, einer Kapsel, eines Pellets, Suppositoriums, einer Lösung, insbesondere einer Injektions- oder Infusionslösung, von Augen-, Nasen und Ohrentropfen, eines Safts, einer Kapsel, einer Emulsion oder Suspension, eines Globuli, Styli, Aerosols, Puders, einer Paste, Creme oder Salbe eingesetzt wird.

15. Verwendung einer Verbindung gemäß mindestens einem der Ansprüche 1 bis 11 zur Herstellung eines Arzneimittels zur Therapie oder Prophylaxe einer kardiovaskulären Erkrankung oder eines thromboembolischen Ereignisses, insbesondere in oraler, subkutaner, intravenöser oder transdermaler Form.

16. Verwendung einer Verbindung gemäß mindestens einem der Ansprüche 1 bis 11 zur Herstellung eines Diagnostikums zur Diagnose eines thromboembolischen Ereignisses.

## Claims

1. A compound of the formula I 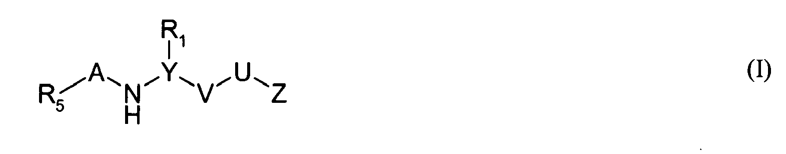 where
A is P₂-P₁ with 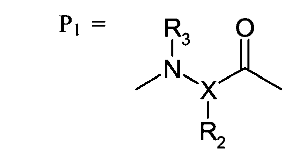 and
P₂ is derived from one of the following amino acids in the D configuration: D-2,3-diaminopropionic acid, D-2,4-diaminobutyric acid, D-citrulline, D-homocitrulline, D-norcitrulline, D-arginine, D-homoarginine, D-norarginine, D-3-guanidinophenylalanine, D-3-guanidinophenylhomoalanine, D-3-guanidinophenylglycine, D-3-amidinophenylalanine, D-3-amidinophenylhomoalanine, D-3-amidinophenylglycine, D-4-guanidinomethylphenylalanine, D-4-guanidinomethylphenylhomoalanine, D-4-guanidinomethylphenylglycine, D-3-guanidinomethylphenylalanine, D-3-guanidinomethylphenylhomoalanine, D-3-guanidinomethylphenylglycine, D-3-amidinopiperidinylalanine, D-3-amidinopiperidinylhomoalanine, D-3-amidinopiperidinylglycine, n-butylamidinoglycine, n-pentylamidinoglycine, n-propylamidinoglycine, D-canavanine, D-homocanavanine, D-norcanavanine, 2-amino-4-amidinohydrazonobutyric acid, 2-amino-3-amidinohydrazonopropionic acid, 2-amino-5-amidinohydrazonopentanoic acid, 2-amino-4-(pyridin-4-ylamino)butyric acid, 2-amino-4-(pyridin-4-ylamino)propionic acid, 2-amino-4-(pyridin-4-ylamino)pentanoic acid, 4-imidazolylpropargylglycine, D-histidine, D-homohistidine, D-histidine-(1-methyl), D-homohistidine-(1-methyl), D-histidine-(3-methyl), D-homohistidine-(3-methyl), D-alanine(4-[5-2(-amino)imidazoyl], D-homoalanine(4-[5-2(-amino)imidazoyl], D-glycine(4-[5-2(-amino)imidazoyl], D-alanine(3-(2-pyrimidinyl), D-homoalanine(3-(2-pyrimidinyl), D-alanine(3-(5-pyrimidinyl), D-homoalanine(3-(5-pyrimidinyl), D-2-amino-3-(2-aminopyrimidin-5-yl)propionic acid, D-2-amino-4-(2-aminopyrimidin-5-yl)butyric acid, D-alanine(3-(2-benzimidazolyl)), D-homoalanine(3-(2-benzimidazolyl)), D-tryptophan substituted by aminoalkyl groups on the indole ring, D-homotryptophan substituted by aminoalkyl groups on the indole ring, D-2-amino-3-(6-aminopyridin-3-yl)propionic acid, D-2-amino-4-(6-aminopyridin-3-yl)butyric acid, D-2-amino-3-(6-amino-2-methylpyridin-3-yl)propionic acid, D-2-amino-4-(6-amino-2-methylpyridin-3-yl)butyric acid, D-2-amino-3-(6-amino-2,4-dimethylpyridin-3-yl)propionic acid, D-2-amino-4-(6-amino-2,4-dimethylpyridin-3-yl)butyric acid, D-3-hydroxyamidinophenylalanine, D-3-hydroxyamidinophenylhomoalanine, D-3-hydroxyamidinophenylglycine,
R₁ is H or -(CH₂)ₐCOOR₆ with a = 0, 1, 2, 3, 4 or 5, where R₆ is a branched or unbranched alkyl radical having 1 to 6 C atoms,;
R₂ is H, a branched or unbranched alkyl radical having 1 to 8 C atoms, or -(CH₂)_{c}COOR₈ with c = 1, 2, 3 or 4, where R₈ is H or a branched or unbranched alkyl radical having 1 to 6 C atoms,, or
-(CH₂)_{d}-OR₉ with d = 1, 2, 3 or 4, where R₉ is H, or -(CH₂)ₑOR₁₀, -(CH₂)ₑSR₁₀, (CH₂)ₑ-guanidino, -(CH₂)ₑ-imidazole or -(CH₂)ₑNHR₁₀ with e = 1, 2, 3, 4 or 5, where R₁₀ is H, a branched or unbranched alkyl radical having 1-16 C atoms or a substituted or unsubstituted aryl, heteroaryl, aralkyl or heteroaralkyl radical, where the alkyl radical has 1 to 16 C atoms, and the aryl or heteroaryl radical has 4 to 14 C atoms;
R₃ is H or -(CH₂)_{b}R₇ with b = 1, 2, 3, 4, 5, 6, 7 or 8, where R₇ is H, a branched or unbranched alkyl radical having 1 to 10 C atoms, or a charged radical;
R₅ is -(CH₂)_{g}(CH₃)ₕ, -(CH₂)ᵢ-aryl with g + h = i = 0, 1, 2 or 3, -SO₂R₁₂, -COR₁₂, or -COOR₁₂, where R₁₂ is a branched or unbranched alkyl having 1-16 C atoms, a substituted or unsubstituted aryl, heteroaryl, aralkyl or heteroaralkyl radical, an adamantyl, a camphor, a cyclohexylmethyl radical,
where R₅ is having a charged or uncharged group;
U is a phenyl or cyclohexyl radical;
a heterophenyl or heterocyclohexyl radical , or
a thiophene radical;
V is (CH₂)ₙ with n = 0, 1, 2 or 3;
X is N or CH;
Y is N or (CH)ₘ with m = 0 or 1;
Z occurs in the 3 or 4 position and is an aminomethyl, a guanidino function or an amidino group 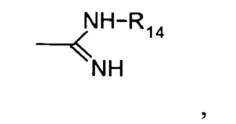 where R₁₄ is H, OH, NH₂, -COR₁₅ or -COOR₁₅, where R₁₅ is a branched or unbranched alkyl radical having 1 to 16 C atoms or a substituted or unsubstituted aryl or heteroaryl, aralkyl or heteroaralkyl radical, where the alkyl radical preferably has 1 to 16 C atoms and the aryl or heteroaryl radical preferably has 4 to 14;
or a compound
where R₅ is 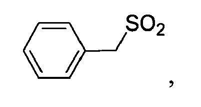 P₁ is 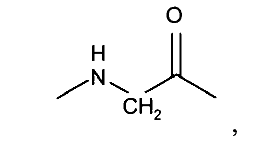 NH-YR₁-V-U-Z is 4-amindinobenzyl amide and P2 is selected from D-Phe(3-amidino) or D-Arg;
**characterized in that** one or more charged radicals are present in the radicals R₁, R₂, R₃ or R₅.

2. The compound as claimed in claim 1, where U is substituted at 1, 2 or 3 positions preferably by a halogen, in particular fluorine or chlorine, or a methyl, ethyl, propyl, methoxy, ethoxy or propoxy radical.

3. The compound as claimed in claim 1 or 2, where a carboxyl group is present protected as ester, preferably as ethyl ester.

4. The compound as claimed in at least one of claims 1 to 3, where R₉ in this case is an alkylcarbonyl, aralkylcarbonyl, alkyloxycarbonyl or aralkyloxycarbonyl radical, where the alkyl radical preferably has 1 to 6, in particular 1 to 4, C atoms and the aryl radical preferably has 5 to 8, in particular 6, C atoms.

5. A compound of the formula I, **characterized in that** the compound has the following structure:

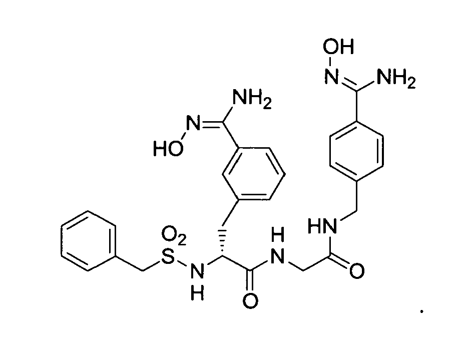

6. The compound as claimed in at least one of claims 1 to 5, **characterized in that** the substituent on the substituted aryl, heteroaryl, aralkyl or heteroaralkyl radical is a halogen, preferably fluorine, chlorine or bromine, in particular fluorine or chlorine.

7. The compound as claimed in at least one of claims 1 to 6, **characterized in that** the compounds are in the form of salts with mineral acids, preferably as hydrochlorides, or preferably as salts with suitable organic acids.

8. The compound as claimed in claim 7, **characterized in that** salts of mineral acids are also sulfates, and suitable organic acids are acetic acid, formic acid, methylsulfonic acid, succinic acid, malic acid or trifluoroacetic acid, with preferred salts of organic acids being acetates.

9. The compound as claimed in at least one of claims 1 to 8, **characterized in that** R₃ is a charged radical with a -(CH₂)ⱼCOOR₁₃, -(CH₂)ⱼSO₂R₁₃, -(CH₂)ⱼNH₂, -(CH₂)ⱼ-amidino, -(CH₂)ⱼ-hydroxyamidino or -(CH₂)ⱼ-guanidino group with j = 0, 1 or 2, where R₁₃ is H or an alkyl radical having preferably 1 to 6 C atoms, in particular 1 to 4, especially ethyl.

10. The compound as claimed in at least one of claims 1 to 9, **characterized in that** R₅ is modified with a charged radical selected from a -(CH₂)ⱼCOOR₁₃, -(CH₂)ⱼSO₂R₁₃, -(CH₂)ⱼNH₂, -(CH₂)ⱼ-amidino, -(CH₂)ⱼ-hydroxyamidino or -(CH₂)ⱼ-guanidino group with j = 0, 1 or 2, wherein R₁₃ is H or an alkyl radical having preferably 1 to 6 C atoms, in particular 1 to 4, especially ethyl.

11. Compound selected from
benzylsulfonyl-dPhe(3-amidino)-Gly 4-(amidino)benzylamide, or
benzylsulfonyl-dArg-Gly-4-(amidino)benzylamide.

12. A method for preparing a compound as claimed in at least one of claims 1 to 11, **characterized in that** the respective amino acids are coupled sequentially to a 4-acetyloxamidinobenzylamine, with the N-terminal amino acid either already carrying the R₅ radical or the latter subsequently being linked thereto.

13. A medicament containing a compound as claimed in at least one of claims 1 to 11 and pharmaceutically suitable excipients and/or additives.

14. The medicament as claimed in claim 13, where the medicament is employed in the form of a tablet, of a coated tablet, of a capsule, of a pellet, suppository, of a solution, in particular of a solution for injection or infusion, of eyedrops, nose and eardrops, of a syrup, of a capsule, of an emulsion or suspension, of a pessary, stick, aerosol, dusting powder, of a paste, cream or ointment.

15. The use of a compound as claimed in at least one of claims 1 to 11 for prepearing a medicament for the therapy or prophylaxis of a cardiovascular disorder or of a thromboembolic event, in particular in oral, subcutaneous, intravenous or transdermal form.

16. The use of a compound as claimed in at least one of claims 1 to 11 for preparing a diagnostica for the diagnosis of a thromboembolic event.

## Revendications

1. Composé de formule générale I 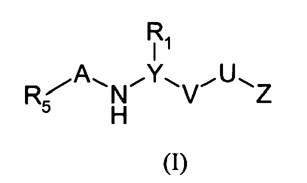 dans lequel
A est P₂ - P₁ et
P₁ = 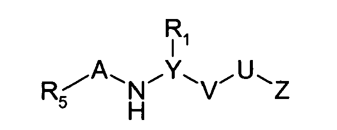
P₂ dérive de l'un des amino-acides suivants en configuration D:
acide D-2,3-diaminopropionique,
acide D-2,4-diaminobutyrique,
D-citrulline,
D-homocitrulline,
D-norcitrulline,
D-arginine,
D-homoarginine,
D-norarginine,
D-3-guanidinophénylalanine,
D-3-guanidinophénylhomoalanine,
D-3-guanidinophénylglycine,
D-3-amidinophénylalanine,
D-3-amidinophénylhomoalanine,
D-3-amidinophénylglycine,
D-4-guanidinométhylphénylalanine,
D-4-guanidinométhylphénylhomoalanine,
D-4-guanidinométhylphénylglycine,
D-3-guanidinométhylphénylalanine,
D-3-guanidinométhylphénylhomoalanine
D-3-guanidinométhylphénylglycine,
D-3-amidinopiperidinylalanine,
D-3-amidinopiperidinylhomoalanine,
D-3-amidinopiperidinylglycine,
n-butylamidinoglycine,
n-pentylamidinoglycine,
n-propyl-amidinoglycine,
D-canavanine,
D-homocanavanine
D-norcanavanine,
acide 2-amino-4-amidino-hydrazonobutyrique,
acide 2-amino-3-amidino-hydrazonopropionique
acide 2-amino-5-amidino-hydrazonovalérianique,
acide 2-amino-4-(pyridine-4-ylamino)-butyrique,
acide 2-amino-4-(pyridine-4-ylamino)-propionique,
acide 2-amino-4-(pyridine-4-ylamino)-valérianique,
4-imidazolyl-propargylglycine,
D-histidine,
D-homohistidine
D-histidine-(1-méthyle),
D-homohistidine-(1-méthyle),
D-histidine-(3-méthyle),
D-homohistidine-(3-méthyle);
D-alanine(4-[5-2(-amino)-imidazoyl]),
D-homoalanine- {4-[5-2(-amino)-imidazoyle]}
D-glycine-{4-[5-2 (-amino)-imidazoyle]},
D-alanine-[3-(2-pyrimidinyle)],
D-homoalanine-[3-(2-pyrimidinyle)],
D-alanine-[3-(5-pyrimidinyle)],
D-homoalanine-[3-(5-pyrimidinyle)],
acide D-2-amino-3-(2-amino-pyrimidine-5-yle) propionique,
acide D-2-amino-4-(2-aminopyrimidine-5-yle) butyrique,
D-alanine-[3-(2-benzimidazolyle)],
D-homoalanine-[3-(2-benzimidazolyle)],
D-tryptophane à substituants aminoalkyles sur l'anneau indole,
D-homotryptophane à substituants aminoalkyles sur l'anneau indole,
acide D-2-amino-3-(6-amino-pyridine-3-yle)-propionique,
acide D-2-amino-4-(6-amino-pyridine-3-yle)-butyrique,
acide D-2-amino-3-(6-amino-2-méthylpyridine-3-yle)-propionique,
acide D-2-amino-4-(6-amino-2-méthyl-pyridine-3-yle)-butyrique,
acide D-2-amino-3-(6-amino-2,4-diméthyl-pyridine-3-yle)-propionique,
acide D-2-amino-4-(6-amino-2,4-diméthylpyridine-3-yle)-butyrique,
D-3-hydroxyamidinophénylalanine,
D-3-hydroxyaminidophénylhomoalanine,
D-3-hydroxyamidino-phénylglycine;
R₁ est un atome d'hydrogène ou -(CH₂)ₐCOOR₆ avec a = 0, 1, 2, 3, 4 ou 5, R₆ étant un radical alkyle ramifié ou non ramifié comportant 1 à 6 atomes de carbone;
R₂ est un atome d'hydrogène, un radical alkyle ramifié ou non ramifié comportant 1 à 8 atomes de carbone, ou
-(CH₂)_{c}COOR₈ avec c = 1, 2, 3 où R₈ est un atome d'hydrogène ou un radical alkyle ramifié ou non ramifié comportant 1 à 6 atomes de carbone, ou
-(CH₂)_{d}-OR₉ avec d = 1, 2, 3 ou 4, où R₉ est un atome d'hydrogène, ou -(CH₂)ₑOR₁₀, -(CH₂)ₑSR₁₀, -(CH₂)ₑ-guanidino, -(CH₂)ₑ-imidazole ou (CH₂)ₑNHR₁₀ avec e = 1, 2, 3, 4 ou 5, et R₁₀ est un atome d'hydrogène, un radical alkyle ramifié ou non ramifié comportant 1 à 16 atomes de carbone, ou bien un radical aryle, hétéroaryle, aralkyle ou hétéroaralkyle, substitué ou non substitué, le radical alkyle comportant 1 à 16 atomes de carbone, et le radical aryle ou hétéroaryle 4 à 14 atomes de carbone;
R₃ est un atome d'hydrogène ou -(CH₂)_{b}R₇ avec b = 1, 2, 3, 4, 5, 6, 7 ou 8, et R₇ est un atome d'hydrogène, un radical alkyle ramifié ou non ramifié comportant 1 à 10 atomes de carbone ou un radical chargé;
R₅ est -(CH₂)_{g}(CH₃)ₕ, -(CH₂)ᵢ-aryle avec g + h = i = 0, 1, 2 ou 3, -SO₂R₁₂, COR₁₂ ou -COOR₁₂, R₁₂ étant un alkyle ramifié ou non ramifié comportant 1 à 16 atomes de carbone, un radical aryle, hétéroaryle, aralkyle ou hétéroaralkyle, substitué ou non substitué, un radical adamantyle, un radical camphre, un radical cyclohexylméthyle, R₅ comportant un groupe chargé ou non chargé
U est un radical phényle ou cyclohexyle, un radical hétérophényle ou hétérocyclohexyle ou un radical thiophène;
V est (CH₂)ₙ avec n = 0, 1, 2 ou 3;
X est N ou CH;
Y est N ou (CH)ₘ avec m = 0 ou 1;
Z apparaît en position 3 ou 4 et est une fonction aminométhyle, une fonction guanidine ou un groupe amidine 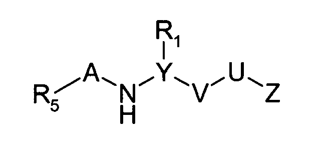 groupe dans lequel R₁₄ est un atome d'hydrogène, OH, NH₂, -COR₁₅ ou COOR₁₅,
R₁₅ étant un radical alkyle ramifié ou non ramifié comportant 1 à 16 atomes de carbone ou un radical aryle ou hétéroaryle, aralkyle ou hétéroaralkyle, substitué ou non substitué, le radical alkyle comportant de préférence 1 à 16 atomes de carbone et le radical aryle ou hétéroaryle de préférence 4 à 14 atomes de carbone;
ou forme un composé avec 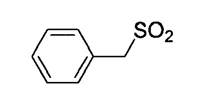 semblable à R₅ 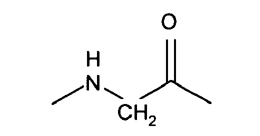 semblable à P₁
NH-YR₁-V-U-Z semblable à 4-amidinobenzylamide et P₂ est choisi entre D-phe(3-amidino) ou D-arg;
ledit composé étant **caractérisé en ce que** un ou plusieurs radicaux chargés sont présents dans les radicaux R₁, R₂, R₃ ou R₅.

2. Composé selon la revendication 1, où U est substitué en 1, 2 ou 3 positions, par un halogène, en particulier le fluor ou le chlore, ou par un radical méthyle, éthyle, propyle, méthoxy, éthoxy ou propoxy.

3. Composé selon la revendication 1 ou 2, où un groupe carboxyle, est protégé sous forme d'ester, de préférence ester d'éthyle.

4. Composé selon l'une au moins des revendications 1 à 3, où R₉ est dans ce cas un radical alkylcarbonyle, aralkylcarbonyle, alkyloxycarbonyle ou aralkyloxycarbonyle, le radical alkyle comportant de préférence 1 à 6 atomes de carbone, en particulier 1 à 4 atomes de carbone, et le radical aryle de préférence 5 à 8, en particulier 6 atomes de carbone.

5. Composé de formule générale I, **caractérisé en ce qu'**il présente la structure suivante:

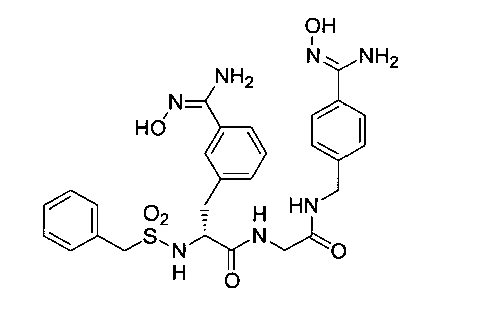

6. Composé selon au moins l'une des revendications 1 à 5, **caractérisé en ce que** le substituant du radical aryle, hétéroaryle, aralkyle ou hétéroaralkyle substitué est un halogène, de préférence du fluor, du chlore ou du brome, en particulier du fluor ou du chlore.

7. Composé selon au moins l'une des revendications 1 à 6, **caractérisé en ce que** les composés se présentent sous forme de sels d'acides minéraux, de préférence sous forme de chlorhydrates ou sous forme de sels avec des acides organiques appropriés.

8. Composé selon la revendication 7, **caractérisé en ce que** les sels d'acides minéraux sont également des sulfates et les acides organiques appropriés sont l'acide acétique, l'acide formique, l'acide méthylsulfonique, l'acide succinique, l'acide malique ou l'acide trifluoracétique, les sels préférés d'acides organiques étant des acétates.

9. Composé selon l'une au moins des revendications 1 à 8, **caractérisé en ce que** R₃ est un radical chargé par un groupe (CH₂)ⱼCOOR₁₃, (CH₂)ⱼSO₂R₁₃, -(CH₂)ⱼNH₂, -(CH₂)ⱼ-amidine, -(CH₂)ⱼ-hydroxyamidine ou -(CH₂)ⱼ-guanidine
dans lequel j = 0, 1 ou 2, et R₁₃ est un atome d'hydrogène ou un radical alkyle comprenant de préférence 1 à 6 atomes de carbone, en particulier 1 à 4, et consistant de préférence en le radical éthyle.

10. Composé selon l'une au moins des revendications 1 à 9, **caractérisé en ce que** R₅ avec un radical chargé est choisi dans le groupe comprenant -(CH₂)ⱼCOOR₁₃, -(CH₂)ⱼ-hydroxyamidine, -(CH₂)ⱼ-guanidine -(CH₂)ⱼ-amidine, -(CH₂)ⱼSO₂R₁₃, -(CH₂)ⱼNH₂, dans lequel j = 0, 1 ou 2 et R₁₃ est un atome d'hydrogène ou un radical alkyle comportant de préférence 1 à 6 atomes de carbone, en particulier 1 à 4, et consistant de préférence en le radical éthyle.

11. Composé consistant en
- benzylsulfonyl-dPHE(3-amidino)-GLY-4-(amidino)-benzylamide
ou
- benzylsulfonyl-dARG-GLY-4-(amidino)-benzylamide.

12. Procédé de fabrication d'un composé selon l'une au moins des revendications 1 à 11, **caractérisé en ce que** les aminoacides correspondants sont couplés séquentiellement à une 4-acétyloxamidinobenzylamine, et que soit l'aminoacide N-terminal porte déjà le radical R₅, soit celui-ci lui est lié ensuite.

13. Médicament contenant un composé selon l'une au moins des revendications 1 à 11, ainsi que des matières consommables et/ou des additifs appropriés du point de vue pharmaceutique.

14. Médicament selon la revendication 13, le médicament étant utilisé sous la forme d'un comprimé, d'une dragée, d'une capsule, d'une pilule, d'un suppositoire, d'une solution, en particulier d'une solution pour injection ou infusion, de gouttes pour les yeux, le nez et les oreilles, d'un sirop, d'une capsule, d'une émulsion ou d'une suspension, de globules, de pointes, d'aérosols, de poudres, d'une pâte, d'une crème ou d'une pommade.

15. Utilisation d'un composé selon l'une au moins des revendications 1 à 11 pour la préparation d'un médicament pour la thérapie ou la prophylaxie d'une maladie cardiovasculaire ou d'un accident thrombotico-embolique, en particulier sous forme orale, sous-cutanée, intraveineuse ou transdermique.

16. Utilisation d'un composé selon l'une au moins des revendications 1 à 11 pour préparer un agent pour le diagnostic d'un accident thrombotico-embolique.
